(19) **Europäisches Patentamt / European Patent Office / Office européen des brevets**

(11) **EP 4 729 602 A1**

(12) **EUROPEAN PATENT APPLICATION**

(43) Date of publication:
**22.04.2026 Bulletin 2026/17**

(21) Application number: **24383134.4**

(22) Date of filing: **15.10.2024**

(51) International Patent Classification (IPC):
**C12N 1/20** (2026.01)         **C12N 9/02** (2006.01)
**C12P 21/02** (2006.01)

(52) Cooperative Patent Classification (CPC):
**C12N 1/20; C12N 9/0089; C12P 21/02;**
C12R 2001/01; C12Y 115/01001

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
• **Universitat Pompeu Fabra**
  **08002 Barcelona (ES)**
• **Fundació Institució Catalana De Recerca I Estudis Avançats**
  **08010 Barcelona (ES)**

(72) Inventors:
• **GÜELL CARGOL, Marc**
  **08002 Barcelona (ES)**

• **SANTOS MORENO, Javier**
  **08002 Barcelona (ES)**
• **NEVOT SÁNCHEZ, Guillermo**
  **08002 Barcelona (ES)**
• **TOLOZA MATURANA, Lorena Viviana**
  **08002 Barcelona (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

Remarks:
The complete document including Reference Table(s) and the Sequence Listing(s) can be downloaded from the EPO website

(54) **ANTIOXIDANT-PRODUCING STRAIN**

(57)    The present invention refers to a composition comprising *Cutibacterium acnes (C. acnes)* bacterial strains, wherein said strains are characterized by being modified to secrete a heterologous protein by insertion of an expression construct comprising at least one or more transcription promoter sequences, one or more ORFs (Open Reading Frames) encoding the heterologous pro-tein, and one or more transcription termination sequences; wherein the heterologous protein is characterized by comprising at the N-terminus of the protein sequence, a secretion signal which is processed by *C. acnes* to secrete the protein, and wherein the heterologous protein is the superoxide dismutase enzyme.

EP 4 729 602 A1

# EP 4 729 602 A1

## Description

### Technical field

The present invention relates to the field of medicine, in particular to an antioxidant-producing strain able to rescue keratinocytes from oxidative stress, preferably UV-induced oxidative stress.

### Background art

The skin is the largest organ in the human body. Beyond its function as a barrier against pathogens or environmental factors, the skin holds relevant physiological functions such as heat regulation or immune surveillance. At the same time, it represents a crucial determinant of our physical appearance and social contact[1]. More than 3000 different conditions affect the skin, with almost one third of the world's population having at least one[2,3]. These diseases have a significant impact on patients' quality of life, ranging from life-threatening (malignant melanoma) to chronic and socially impairing conditions (psoriasis, atopic dermatitis (AD) or acne)[3]. Environmental factors also influence skin health. For instance, excessive UV exposure causes premature ageing and skin cancer[4]. Current treatments against the most frequent chronic skin conditions such as AD or psoriasis focus on symptom control and often cause side effects[5,6]. Novel treatments that target the underlying immune components of these diseases are effective, but they pose a significant economic burden in national healthcare systems[7] - e.g. dupilumab, a monoclonal antibody used for AD that blocks IL-4 and IL-13, has a yearly cost of more than 30,000$ per patient[8].

Engineered live biotherapeutic products (eLBPs) aim to combine the natural adaptation of human microbes to their host[9] with the synthetic design of sensors, circuits, and actuators that enhance the capabilities of those microbes. While some eLBPs have already shown encouraging results treating metabolic diseases[10,11], infections[12] or cancer[13] in the gut or even the lung[14,15], there are still few instances of skin eLBPs. The most promising examples focus on engineering lactic acid bacteria or *Staphylococcus epidermidis* for accelerated wound healing[16] or as cancer vaccines[17] and mosquito repellent[18], with promising preliminary results in mice. These bacteria might produce transient benefits, but are suboptimal as a long term skin-resident eLBP. Lactic acid bacteria are not native skin residents, and their engraftment is low. Despite being ubiquitous in the skin, *S. epidermidis* shows a lower relative abundance and a higher turnover compared to other skin microbes like *C. acnes*[19], and might incur in horizontal gene transfer with related pathogens like *S. aureus*[20].

*Cutibacterium acnes* is the most abundant skin commensal[21]. This Gram-positive and facultative anaerobic bacterium predominantly colonizes the pilosebaceous units, where it metabolizes sebum lipids[22]. This specialized ecological niche constitutes a privileged location for eLBP deployment. The hair follicle is a physiological hotspot relevant for immune education, and the main route for topical absorption of therapeutics[23]. The turnover of *C. acnes* in the skin is low[19], and genomic studies report virtually monoclonal populations within pilosebaceous units[24], with high genome stability across strains[25]; the low competition and low recombination events are relevant features regarding biosafety[26]. In addition, *C. acnes* can be transplanted from donor to recipient skin using topical applications, which has been shown to modulate the relative abundance of recipient *C. acnes* strains for up to several weeks[27]. Taken together, *C. acnes* is an ideal chassis for skin eLBP development due to its location, high abundance, relevance in healthy skin, colonization capabilities and low turnover, as we demonstrated recently through a sebum modulation application[28]

### Brief description of the figures

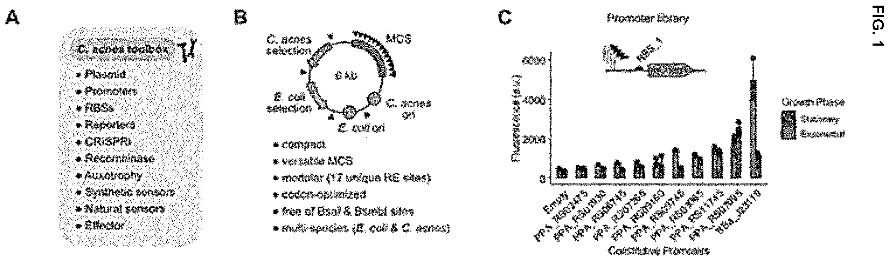

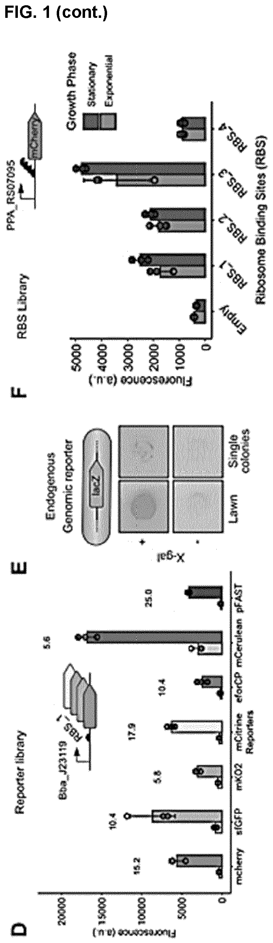

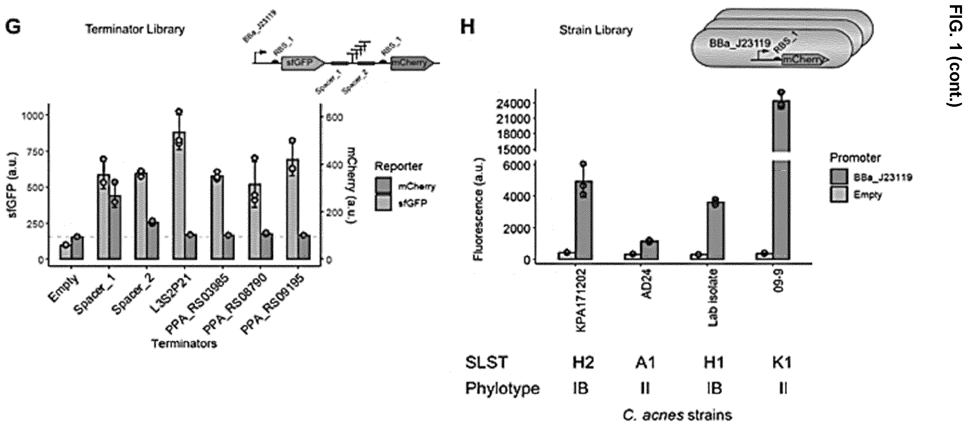

Figure 1: Development of a toolbox for C. acnes genetic engineering. (A) List of all tools developed in this study. (B) Schematics of C. acnes replicative plasmid optimizations (C) Average of population median mCherry fluorescent values of three independent replicates for nine different constitutive endogenous promoters, a synthetic consensus promoter (BBa_J23119) from E. coli and a control harbouring a no insert plasmid (Empty) for exponential and stationary growth phases. (D) Average of population median fluorescent values for C. acnes harbouring a fluorescent reporter (coloured bars) compared with a no insert plasmid control (grey bars). Values depicted above bars indicate average fold change of three independent replicates. (E) Pictures from C. acnes KPA171202 wild-type strain grown either as a lawn or as single colonies in a brucella media agar plate supplemented or not with X-gal. (F) Average of population median mCherry fluorescent values of three independent replicates for four different RBSs driving mCherry translation when expressed with the constitutive promoter PPA_RS07095 compared with an no insert plasmid control (Empty) for exponential and stationary growth phase. RBS_1 and RBS_2 correspond to strong E. coli RBSs with the conserved AGGAGG motif whereas RBS_3 and RBS_4 are de novo RBS designed with the RBS calculator [29]. (G) Average of population median mCherry (red) and sfGFP (green) fluorescent values of three

2

independent replicates for five different transcriptional terminator sequences: the natural E. coli terminator ECK120033737, the synthetic terminator L3S2P21, and three endogenous terminator sequences from C. acnes. sfGFP and mCherry are located in the same transcriptional unit and the terminator is placed in-between. The whole transcriptional unit is expressed using the BBa_J23119 promoter and values are compared to a strain harbouring a no-insert plasmid control (Empty) and two control strains with spacer sequences (Spacer_1 or Spacer_2) but no terminator. The red dashed line indicates the mCherry fluorescent value for the no-insert plasmid control (Empty). (H) Average of population median mCherry fluorescent values of three independent replicates for four different C. acnes strains expressing mCherry controlled with the BBa_J23119 constitutive promoter (red) compared with the same strain harbouring a no insert plasmid (Empty, grey). The table below indicates the C. acnes Sequence Locus Sequence Typing (SLST)[30] or the recA-based phylotype[31] for each of the strains.

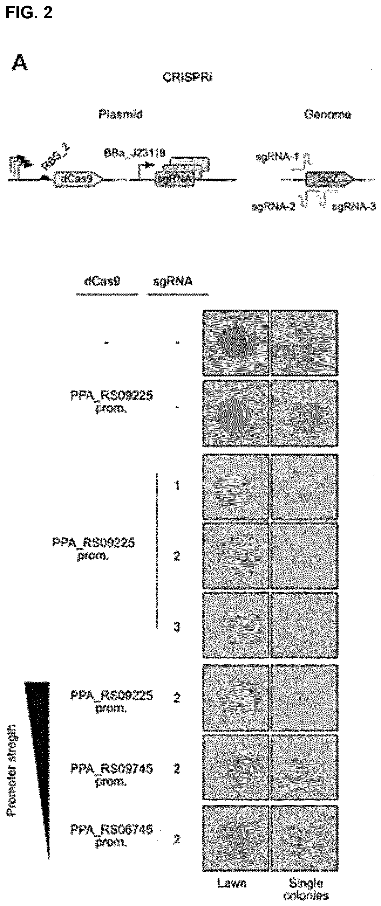

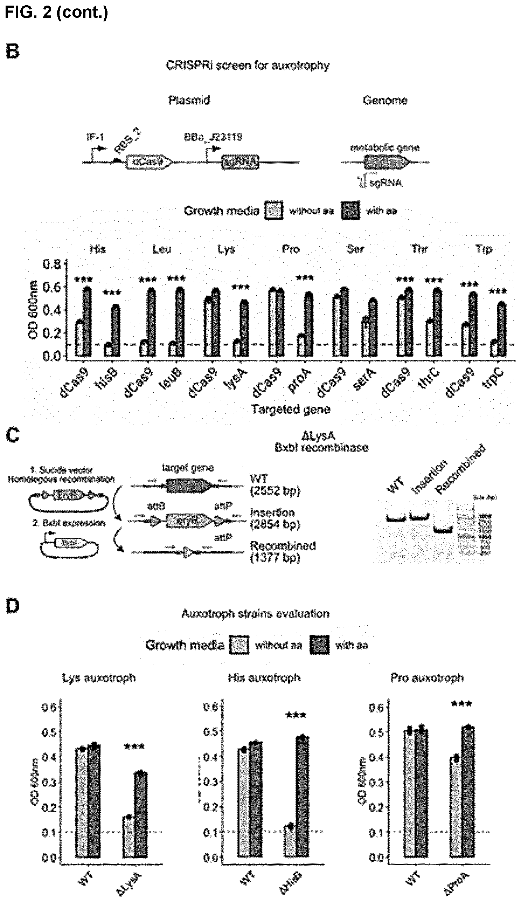

**Figure 2:** CRISPR interference for quick prototyping of auxotrophies for biocontainment. (A) dCas9 and gRNA optimization against the genomic reporter lacZ (PPA_RS08600). Pictures show the absence or presence of blue colour in C. acnes lawn or single colonies grown in Brucella media agar plates supplemented with X-gal. (B) CRISPRi screen of C. acnes metabolic genes potentially leading to a single-gene knock-out amino acid auxotrophy based on homology. Optical density at 600nm (OD600) of C. acnes cultures grown for one day in the presence or absence of a specific amino acid compared to a control (dCas9) without gRNA cassette (i.e. without gRNA and its corresponding promoter). For instance, to evaluate His auxotrophy, the basal medium was supplemented with all amino acids except histidine and growth was measured both in the absence and presence of histidine for the auxotroph and dCas9 control. The dashed grey line indicates the starting culture OD600 at 0.1. Statistical significance was determined using a Student's t-test to compare the strain when grown in the presence and absence of the studied amino acid. A p-value < 0.001 is denoted with three stars (***). (C) Agarose gel showing the PCR amplification of the lysA locus from a wild-type strain (WT), a strain with an erythromycin cassette insertion flanked by BxbI recombination sites (Insertion) and a third strain in which the erythromycin cassette has been removed through BxbI expression (Recombined). The diagram shows the process to obtain such as a strain and the expected size for each locus amplification. (D) Growth of a ∆lysA, ∆proA and ∆hisB knock-out strains compared to wild-type (WT) C. acnes in the presence or absence of Lysine, L-Proline or L-Histidine after four days of incubation. The dashed grey line indicates the starting culture OD600, 0.1. Statistical significance was determined using a Student's t-test to compare the strain when grown in the presence and absence of the studied amino acid. A p-value < 0.001 is denoted with three stars (***).

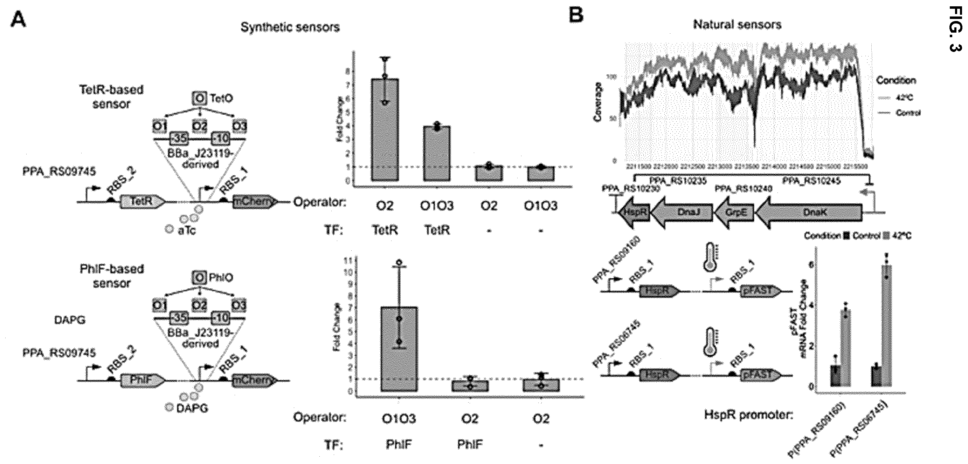

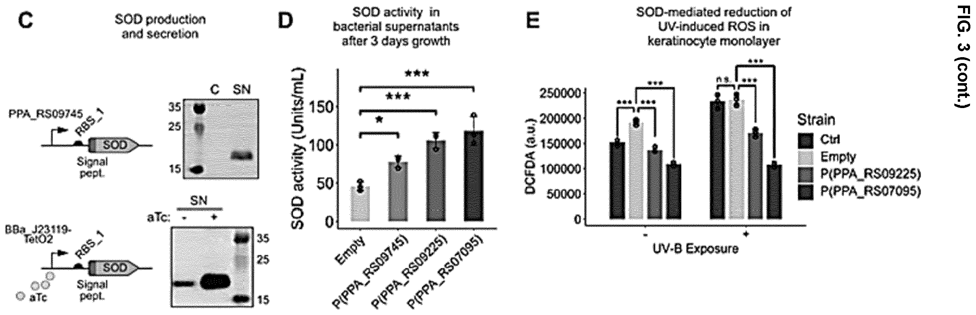

**Figure 3:** Engineered sensing and actuation in C. acnes. (A) Schematics of a synthetic BBa_J23119-based promoter including operator sequences from the TetR and PhlF transcriptional repressors. Fold-change mCherry population median fluorescence upon induction, for different promoter architectures, in the presence or absence of the corresponding transcription factor. (B) Adaptation of temperature-responsive C. acnes endogenous genes for temperature sensing. The graph on top displays the coverage of RNA-seq reads for a specific genome region containing the differentially expressed temperature-responsive operon. The diagrams depict the plasmid design of two temperature sensing plasmids, both carrying the temperature-sensitive HspR transcription factor regulated by two distinct constitutive promoters (PPA_RS09160 and PPA_RS06745, respectively), alongside the temperature-responsive promoter controlling the pFAST reporter. The graph on the bottom right displays the average mRNA fold change increase in these two temperature sensing strains upon 42 °C heat shock treatment measured by RT-qPCR. (C) Top: Western blot detection of SodC-F1 constitutively produced and secreted to the supernatant (labelled as SN) by an engineered C. acnes strain, compared to the cellular (labelled as C) fraction of the same strain. Bottom: Western blot detection of aTc-inducible SodC-F1 production. Shown are supernatant (SN) fractions of uninduced (-) and induced (+) cultures. (D) Superoxide dismutase activity measured in the supernatant of three different SodC-F1-producing strains with different constitutive promoter strengths (P(PPA_RS09745), P(PPA_RS09225) and P(PPA_RS07095) ) compared to a no insert plasmid control (Empty) (E) Reactive oxygen species (ROS) levels measured with CM-H2DCFDA fluorescent dye for N/TERT-2G keratinocytes cells exposed or not to UV-B light. Cells were treated with the supernatant of strains producing SodC-F1 constitutively (from P(PPA_RS09225) or P(PPA_RS07095) promoter) or with the supernatant of a no-insert plasmid control (Empty).

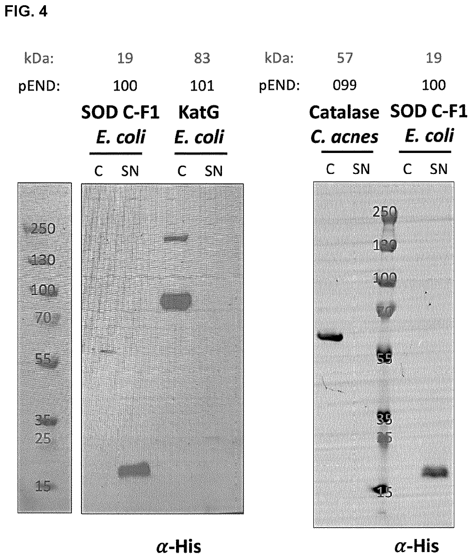

**Figure 4.** Western blot analysis of synthetic C. *acnes* cells engineered to produce and secrete proteins with anti-ROS activity. Both the cellular fraction (C) and the supernatant fraction (SN) were assessed for the presence of the desired proteins: the E. coli KatG catalase (left panel), the C. acnes catalase (right panel), and the E. coli O157:H7 SodC-F1 (both panels). Despite the use of the same promoter, RBS and signal peptide sequences for all three proteins, only the SodC-F1 was secreted to the supernatant, unlike the two catalases.

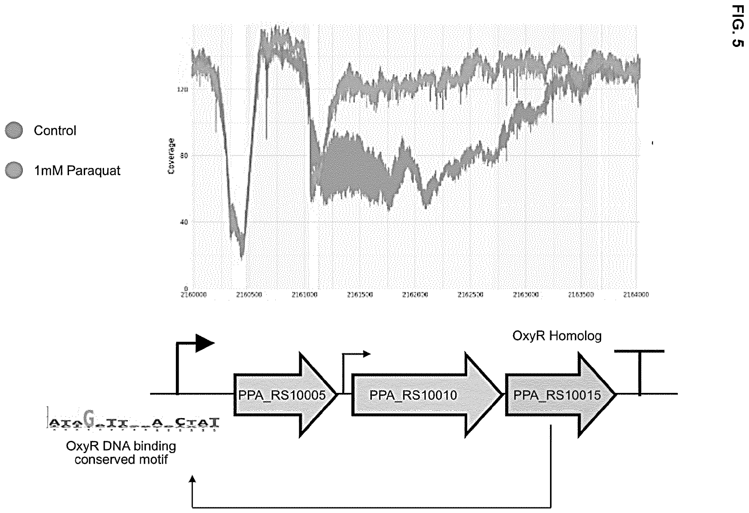

**Figure 5.** The graph displays the coverage of RNA-seq reads for a specific genome region containing the differentially expressed ROS-responsive operon. The scheme depicts de genomic organisation. The sequence logo displays the conservation of the OxyR binding motif compared with the OxyR motif from *E. coli.*

**Description of the embodiments of the invention**

In the present invention, we establish an enabling toolbox for the traditionally intractable C. *acnes* to promote the development of microbiome-based skin therapies (Fig. 1A). We demonstrate the use of these tools to discover auxotrophies and to create an antibiotic marker-free strain for safe deployment in the skin. Moreover, we design a set of natural and synthetic inducible systems for the exogenous or environmental activation of gene expression. As a proof of concept for potential therapeutic applications, we create an antioxidant-producing strain able to rescue keratinocytes from oxidative stress, preferably UV-induced oxidative stress.

To construct this toolbox, we optimized a plasmid from *Propionibacterium freudenreichii* previously described to replicate in *C. acnes*[28,32,33] (Fig. 1B). First, we removed all non-essential sequences as well as many restriction sites commonly used in classical or Golden Gate cloning, reducing the total vector size by more than 2 kb. Additionally, we adapted the plasmid for a modular cloning scheme based on isothermal (Gibson) assembly that allows us to standardize all our genetic parts with common suffix and prefix sequences for quick and efficient cloning[34]. We also standardized and codon optimized two antibiotic resistance cassettes - based on the *ermE* gene from *Saccharopolyspora erythraea* and the *cmx* gene from *Corynebacterium glutamicum* - and used one or the other as the *C. acnes* selection marker. The plasmid backbone contains two origins of replication (one for *E. coli,* the other for *C. acnes)* and two resistance cassettes (one for each of the bacterial hosts). To facilitate the straightforward replacement of those components, we flanked them with unique restriction sites.

We wanted to develop a collection of genetic parts enabling us and others to control and program the behaviour of *C. acnes.* To this goal, we first tested a set of 9 endogenous constitutive promoters that we previously identified by RNA-seq (see Methods) spanning a wide range of expression levels, with most functioning in both exponential and stationary growth phases (Fig. 1C). We also tested a synthetic *E. coli* consensus promoter (BBa_J23119) because of its similarity to the *C. acnes* -10 consensus box[35]. Interestingly, this promoter yielded the highest expression among those tested (Fig. 1C), and was thus used to validate the suitability of 7 fluorescent proteins as *C. acnes* reporters, including the anaerobic fluorescent tag pFAST[36]. Using flow cytometry, we observed an increase in fluorescent signal for all reporters when compared with an empty-plasmid control (Fig. 1D), and we selected mCherry for subsequent experiments due to its reported suitability in anaerobic bacteria after 2h maturation in aerobic conditions[37,38]. We also validated the use of the endogenous copy of *lacZ* - encoding beta-galactosidase - as a genomic reporter in the presence of X-gal (Fig. 1E). We next tested the strength of 4 different ribosome binding sites (RBS): two of these (RBS_3 and RBS_4) were designed using the RBS calculator[29], while the other two (RBS_1 and RBS_2) were strong RBS sequences from *E. coli* - indeed, the rRNA stretch that recognizes the Shine-Dalgarno sequence is only one nucleotide different between *C. acnes* and *E. coli.* Interestingly, the *E. coli* RBS sequences yielded the highest levels of mCherry production (Fig. 1F).

We next measured the transcriptional termination of five sequences: two strong terminators reported to work in E. coli (a natural one and a synthetic one)[39] and three C. acnes endogenous sequences selected from RNA-seq. We used a dual reporter system where sfGFP and mCherry were transcribed together but are separated by the evaluated terminator sequence (Fig. 1G). All five terminators yielded mCherry levels that were reduced with respect to non-terminator spacer controls and with terminator efficiencies ranging from 70% to 90% compared with the Spacer_1 control (Fig. 1G). Adults have a unique mixture of C. acnes strains[21]. Ideally, the engineering of different C. acnes strains could enable strain-specific eLBPs tailored to the specific patient microbial profile, therefore increasing engraftment. For this reason, we wondered how suitable our optimised plasmid and molecular tools were for other C. acnes strains spanning across two phylotypes[31] and four different Sequence Locus Sequence Typing (SLST) types[30]. These strains were able to incorporate our optimized plasmid and to express mCherry controlled by the BBa_J23119 synthetic promoter in exponential phase (Fig.1H).

We used these basic genetic parts to further develop more advanced tools. To interrogate the function of endogenous genes, we sought to validate the use of CRISPR interference (CRISPRi) in *C. acnes.* We had observed that *C. acnes* is naturally able to degrade the synthetic compound X-gal to yield blue-coloured colonies (Fig. 1E), and we hypothesized that this β-galactosidase activity was due to a genomic *lacZ* homolog, PPA_RS08600. We designed single guide RNAs (sgRNAs) against the 5' end of the *lacZ* coding sequence, and we combined them with different expression levels of dCas9. Designs featuring high expression levels of dCas9 yielded colourless colonies in the presence of X-gal regardless of the sgRNA used, indicating an efficient repression of endogenous *lacZ* (Fig. 2A).

Next, we focused on designing a biocontainment strategy for *C. acnes* that ensures the safe deployment of therapeutically relevant strains. Our goal was to generate an auxotrophic strain without any heterologous antibiotic resistance gene. Due to the low genome integration efficiency in *C. acnes*[28], we initially turned to plasmid-borne CRISPRi to screen for genes that would render *C. acnes* auxotrophic upon knock-out. We targeted 8 different putative gene candidates whose individual disruption would lead to an amino acid auxotrophy (Fig. 2B), based on their homology to auxotrophic strains described in the *E. coli* KEIO collection[40]. We then evaluated which of the CRISPRi knock-downs impaired bacterial growth when the amino acid was not supplied in a rich defined custom medium (See methods). Lysine and proline biosynthesis pathway repression showed the highest growth defect compared to a dCas9-only control (Fig. 2B). Histidine, leucine, and tryptophan pathway repression also showed growth defects, but growth was also reduced in the dCas9-only control, suggesting that the wild-type expression of the targeted genes cannot compensate for the absence of those amino acids in the medium (Fig. 2B). Interestingly, we did not manage to knock down the cysteine pathway, likely due to the relevance of this amino acid for *C. acnes*[41].

In the light of these results, we generated *C. acnes ΔlysA, ΔproA* and *ΔhisB* strains by using a homologous recombination cassette harbouring the erythromycin resistance gene *ermE* (Fig. 2C). To enable the removal of the genome-integrated antibiotic resistance gene, we flanked *ermE* with the *attP* and *attB* recombination sites specific for the Bxbl serine integrase. Constitutive expression of the Bxbl gene from a replicative plasmid selected with a different antibiotic resistance marker (*cmx*) resulted in the successful removal of the complete *ermE* gene for all tested colonies after transformation (Fig. 2C). After plasmid curation, we obtained three strains without any heterologous antibiotic resistance that preserved the corresponding gene deletions. The lack of lysA and hisB was enough to impair the growth of C. acnes after four days of incubation in the absence of lysine or histidine, respectively (Fig. 2D). In the case of histidine absence, the wild type had reduced growth after 24 h, as observed previously in the CRISPRi screening, but achieved a similar growth level to the histidine-supplemented culture after four days of growth, in contrast to the ΔhisB strain (Fig. 2D). We observed a similar behaviour for lysine supplementation, where the wild-type experienced a slower growth but recovered within four days, while the ΔlysA strain did not show any growth during that time. Regarding the proline auxotroph, proA deletion did not fully suppress growth, but yielded a growth delay in the absence of proline that might impact its ability to compete with other skin-resident microbes and therefore be enough for biocontainment within the natural niche - the human skin (Fig. 2D). While the concentration of free Lys, His and Pro in corneocytes appears to be low on the skin surface[42], a more biologically relevant and tissue realistic skin model is needed to further evaluate the performance of these auxotrophies as a real-world biocontainment strategy.

One of the key benefits of eLBPs is their ability to detect host or environmental signals and to dynamically produce relevant molecules with therapeutic potential. To build such a dynamic "smart" probiotic, we engineered different transcriptional sensors in *C. acnes* following two main strategies: the transplantation of known transcription factors (TFs) from other organisms, and the *de novo* discovery of natural genetic resources from *C. acnes.* For the first approach, we decided to focus on two well-known repressors commonly used in model bacteria, TetR and PhlF. We designed different inducible promoters by placing one or more operator sequences (*tetO* or *phlO*) upstream, downstream or between the -35 and -10 boxes of BBa_J23119, and we combined the resulting promoters with different expression levels of the corresponding TFs (Fig. 3A). In the presence of the inducer (aTc for TetR, DAPG for PhlF), some of the designs yielded inducible mCherry expression of up to ~7-fold compared to the non-induced control (Fig. 3A). While sensors in model bacteria can display large dynamic ranges (of up to three or four orders of magnitude in some cases) as a result of numerous optimizations throughout years of research, the fold-induction of TFs used in non-model bacteria is generally more modest, often in the range of one order of magnitude[43] - e.g. ten-fold for GlnR in Pseudomonas putida[44], seven-fold for FNR in Synechocystis sp. PCC 6803[45], or 5-fold for AceT in Rhodococcus opacus[46].

To discover and co-opt the natural transcription factors already present in the C. *acnes* genome, we performed an RNA-seq on *C. acnes* upon temperature shock, which represents an environmental signal with potential relevance in the skin context. Indeed, temperature change-detection has been previously used to trigger the self-destruction of bacteria leaving the body (i.e. kill switch) for biocontainment[47], or as an activation mechanism induced by ultrasounds[48]. We exposed *C. acnes* to a 42°C heat shock for 15 minutes and we looked for upregulated genes with RNA-seq. From Hidden Markov Models (HMM) models and operon structure conservation, we identified a conserved DnaK-GrpE-DnaJ-HspR chaperon operon well-known in other bacteria to regulate protein refolding in heat-shock stress (Fig. 3B). Specifically, the HspR transcriptional regulator is involved in the regulation of the operon [49,50]. we thus cloned the regulatory sequence together with the constitutively expressed HspR in our replicative plasmid. We tested two plasmid constructs differing only in the strength of the constitutive promoter used to express HspR (Fig. 3B). We measured the pFAST reporter mRNA expression by RT-qPCR after a 42°C heat-shock and observed a 4 and 6-fold increase at the mRNA levels, respectively (Fig. 3B). The higher fold-change observed higher fold-change observed when HspR is expressed from the P(PPA_RS06745) promoter is likely due to the greater abundance of the repressor HspR due to its stronger constitutive promoter. Typically, higher repressor concentrations decrease basal expression levels and result in a greater change in expression upon induction[51].

Ideally, eLBPs should have the dual ability not only to sense the environment but also to produce proteins or molecules with therapeutic relevance directly on the appropriate biological niche. We therefore engineered an antioxidant-secreting *C. acnes* strain for reducing oxidative stress in the skin. Superoxide dismutases (SODs) are enzymes that neutralize the superoxide anion ($O_2^-$) and protect cells from oxidative damage. Among these enzymes, the SodC-F1 from the enterohemorrhagic *E. coli* O157:H7 has shown to be functional in the periplasm, stable against proteases and with high catalytic activity, representing one of the principal defence mechanisms of this bacterium against Reactive Oxygen Species(ROS)[52]. Any type of unstable molecule that contains oxygen and that easily reacts with other molecules in a cell eventually causing oxidative damage to key cellular elements like DNA or RNA is considered a ROS.

We hypothesized that expressing this enzyme in *C. acnes* could reduce ROS levels. To prove this, we first coupled the enzyme with a secretion signal from a highly secreted endogenous protein, RoxP[53,54]. Indeed, the protein was correctly produced and secreted to the supernatant (Fig. 3C), and it maintained its superoxide dismutase activity in the supernatant (Fig. 3D). We also demonstrated the usefulness of our newly-developed transcriptional sensors by constructing a strain that produces and secretes SodC-F1 in an aTc-inducible manner (Fig. 3C). We next challenged the immortalized keratinocyte cell line N/TERT-2G[55,56] with UV-B to induce the formation of ROS. Thereafter, we treated the cells with *C. acnes* supernatants and quantified ROS levels using the fluorescent dye CM-H2DCFDA. The SodC-F1 produced by *C. acnes* was able to significantly reduce UV-mediated ROS levels in keratinocytes (Fig. 3E). The reduction correlated with the strength of the promoter regulating SodC-F1 expression, suggesting that we are able to adjust the antioxidant activity of the engineered strain to match the requirements of the final application. Importantly, the secretion of SodC-F1, which is key for delivering its antioxidant activity, seems to correlate with its small molecular size, since larger antioxidant proteins like the *E. coli* KatG or the *C. acnes* catalase remain in the cellular fraction and are not secreted despite the use of the same promoter, RBS and signal peptide sequence as for SodC-F1 (Fig. 4).

Moreover, deployments of this antioxidant eLBP could incorporate inducible SOD expression, either through artificial inducers like aTc (Fig. 3C), or in response to environmental triggers, such as UV-damage markers produced by keratinocytes. For instance, a bacterial ROS sensor (such as OxyR[57]) controlling the expression of SOD depending on the ROS produced by keratinocytes upon UV exposure would result in an adaptive antioxidant eLBP. Such sensors have been previously reported for the gut environment within an *E. coli* bacterial chassis to detect oxidative stress *in situ*[58]. In *C. acnes,* a putative oxidative stress sensor is homologous to the OxyR transcription factor from *E. coli*. Homologous genes in the OxyR regulon are also present in *C. acnes* and are induced in the presence of oxidative stress generated with the common toxic pesticide paraquat (Fig. 5). The regulatory sequences of these promoters could be taken together with the OxyR transcription factor homolog to induce gene expression in *C. acnes* with ROS, as it was previously done with the original OxyR sensor from *E. coli*[57].

In conclusion, we have established *C. acnes* as an amenable chassis for eLBPs development with potential applications in the skin. We describe a versatile toolbox for its engineering, as well as an auxotrophic strain and antibiotic-free methodologies for genetic engineering towards its safe application in the skin. We demonstrate the detection of both artificial and environmental cues by engineered *C. acnes,* and we provide a proof-of-concept demonstration of oxidative stress reduction on keratinocytes. Taken together, our results pave the way for the use of *C. acnes* for microbiome-based skin therapies.

Therefore, the instant disclosure relates to compositions of *C. acnes* and methods of secreting therapeutically active proteins, preferably superoxide dismutase enzyme, for the treatment of diseases or disorders. The recombinant bacteria disclosed herein are capable of high yield production of functionally active superoxide dismutase enzymes, which are secreted as active polypeptides, preferably as therapeutically active polypeptides. Thus, a first aspect of the invention refers to a composition comprising *Cutibacterium acnes (C. acnes)* bacterial strains, wherein said strains are characterized by being modified to secrete a heterologous protein by insertion of at least an expression construct comprising at least one or more transcription promoter sequences, one or more ORFs (Open Reading Frames) encoding the heterologous protein, one or more transcription termination sequences and preferably one or more RBS (Ribosome Binding Site); wherein the heterologous protein is characterized by comprising at the N-terminus of the protein sequence, a secretion signal which is processed by *C. acnes* to secrete the protein, and wherein the heterologous protein is the superoxide dismutase enzyme.

In some embodiments, the genetically engineered or modified *C. acnes* of the disclosure comprises a gene that is operably linked to a promoter that is not associated with said gene in nature. For example, in some embodiments, the recombinant bacteria disclosed herein comprise a gene that is operably linked to a directly or indirectly inducible promoter that is not associated with said gene in nature.

As used herein, the term "coding region or encoding" refers to a nucleotide sequence that codes for a specific amino acid sequence.

A regulatory region "operably linked" refers to the association of nucleic acid sequences on a single nucleic acid fragment so that the function of one is affected by the other. A regulatory element is operably linked with a coding sequence when it is capable of affecting the expression of the gene coding sequence, regardless of the distance between the regulatory element and the coding sequence. More specifically, operably linked refers to a nucleic acid sequence, e.g., a gene encoding an effector molecule, that is joined to a regulatory sequence in a manner which allows expression of the nucleic acid sequence, e.g., the gene encoding the effector molecule described herein. In other words, the regulatory sequence acts in cis. In one embodiment, a gene may be "directly linked" to a regulatory sequence in a manner which allows expression of the gene. In another embodiment, a gene may be "indirectly linked" to a regulatory sequence in a manner which allows expression of the gene. In one embodiment, two or more genes may be directly or indirectly linked to a regulatory sequence in a manner which allows expression of the two or more genes. A regulatory region or sequence is a nucleic acid that can direct transcription of a gene of interest and may comprise promoter sequences, enhancer sequences, response elements, protein recognition sites, inducible elements, promoter control elements, protein binding sequences, 5 'and 3 'untranslated regions, transcriptional start sites, termination sequences, polyadenylation sequences and introns. A "promoter" as used herein, refers to a nucleotide sequence that is capable of controlling the expression of a coding sequence or gene. Promoters are generally located 5' of the sequence that they regulate. Promoters may be derived in their entirety from a native gene or be composed of different elements derived from promoters found in nature, and/or comprise synthetic nucleotide segments. Those skilled in the art will readily ascertain that different promoters may regulate expression of a coding sequence or gene in response to a particular stimulus, e.g., in a cell- or tissue-specific manner, in response to different environmental or physiological conditions, or in response to specific compounds. Prokaryotic promoters are typically classified into two classes: inducible and constitutive. A "constitutive promoter" refers to a promoter that allows for continual transcription of the coding sequence or gene under its control. Some non-limiting examples of promoters useful in the present invention are HspR C. acnes (PPA_RS10230), HspR Operon promoter temperature sensor from C. acnes (P(PPA_RS10245), OxyR C. acnes promoter ROS Sensing 1 (P(PPA_RS10005)), OxyR C. acnes promoter ROS sensing 2 (P(PPA_RS10010)), or OxyR C. acnes transcription factor (PPA_RS10015):

HspR C. acnes (PPA_RS10230) (SEQ ID NO 18):

ATGGTGAGGCGCAGCAGGAATCTATCCGACGCTGTTGATCTTGCGGTGATCGACA
AGGACGCTGCCATCTTCGCCATCTCAGTGGCGGCAGAGCTCGCCGGAATGCACC
CTCAGACCCTGCGCACTTACGATCGGCTCGGGCTTGTAGTGCCCGAACGCACTCG
GGGGCGGGGGCGTCGCTACTCGATGCGTGACGTTGCTGCGTTGCGGATGGTCCA
GCATCTGTCCCAGGAAGAGGGGATTAACCTCAACGGCATCCGAAGGATCCTTCAG
ATGGAACGTAGGATCGAACAGTTGTCGGAGCAGGTGGACGAGCTCACCGACACC
GTGCGTGGGATGCAAGTGGCGCGTTACCAGGGCAGTGAAGATCCGAGAGTCTTTA
CTGCTGAGTCGACCGGCCGGGTGCATATGGGTCGGACGGTCGTTCATGCCCAGTT
GGCACTGCCTTCTCATCGTGGTTGA

HspR Operon promoter Temperature sensor from C. acnes (P(PPA_RS10245) (SEQ ID NO 19):

CGTGAAGTGCGGAAGCTGCCGGACGGTTCGCCGTCCGGCATTTTTCATGGTCGGA
AGGGGTCGTCTGAGGATGTCTTGACGAGACTTTCGGTGGGTGGGAATACTCCCGC
CGGATCGTCGGTTGAGTGTAGCGAACTCAACTTCATGAAAAATCAGCCAACGTCAA
CTTGAGTGCTCGTGACTCAGGGTCTAGAGTTGAGTCGAAGTCGCTCAAGGGGATG
TAGTCCCAGAACATTCGTCCATCAGGAGG

OxyR C. acnes promoter ROS Sensing 1 (P(PPA_RS10005)) (SEQ ID NO 20):

GGTTAAGGCCGCCCAATACATCTACACCCACCCAGGCGAGGTGTGCCCCGCCAA
GTGGGAAGAAGGCGATGAGACCCTCGCCCCGTCGATCGACCTCGTCGGCAAGAT
CTGA

OxyR C. acnes promoter ROS sensing 2 (P(PPA_RS10010)) (SEQ ID NO 21):

GACCATGGGGGGCATCATGCCACGGGTGCGGGCCCTGGCGACACAGAGAGTCCCG
AGTTCATGATCTTTATAGCTATTGACTATAGATGCTGGTTTAGTGATAGCCTTGATC
TATCACATCGTCGACGAGAAAGGACACCC

OxyR C. acnes transcription factor (PPA_RS10015) (SEQ ID NO 22):

GTGAACCTTCATGATCTGCGGTATTTCGTCGCCCTGGCCGAAGAACACCACTTCG

GCCGGGCTGCGGCGTCATGCGGTGTCAGCCAGCCCACCCTCTCAACTCAAATCCA

CCGGCTCGAGAACGAGCTCGGCGTGGTGCTGGCCGTGAGGGTGGGACGACGCA

TCGAGATCACCCCAGTGGGGGAACGCATCGCCCAAGCTGCCCGTGACATGTTGGT

GCTATCCGACGACATCCGGACCATGGCGCGGGAGGAAGCCGACCGGATGACCCT

CCAGCTGAGGGTCGGGGTATTCCCCACCTTAGGGCCGTTCGTGTTGCCTCAGGTG

ATCGCCCGATGTGACCAGCACGAGCCTGGCGTGAACATCCATATCGTCGAGAAGC

GCACGGCAGAGCTGTTGGAGGCAATCCGCAACGGACAGTTAGACGTCGCCGCAG

TGGCAGCCCCCGTTAATGATGACTCTTTGGTGACTATTCCCGTTTTTCGGGAGGAG

TTCTTGCTCGTGACGGGGGGCTGACGATGAGTTGGCCCGTGAGGAACGACCAATCA

ATCCGCACGACGTTCCCACCGAAGGGCTCATCCTCATGTCGGAGGGACACTGTCT

AAGAGACCAGGTGCTTGAGGTGTGCAGCCCGTCTCATCCGCTGCCTCCCGACACC

CTCACGGCGGGATCCCTCGAGACGCTCCGCGAACTCGTATCGGTGGGCGCCGGT

CAAACCCTCATGCCGCGCAGTGCGATCTCGGCACCACTTCATGCGGACCCGCGCA

TCGTGGTGCGGGAGTTCACCCACCCCAGACCCCACCGCGACATCGTGGTGGCTG

GCAGACCGGCCACAATGCACCGTCCGGCGGTGCAGACCCTGACCACCATCCTGC

GGCAGCTACCAGGTGACGTCGTCGAGCCTCTCGGCGTTGCCGGAGCATCGTTGT

GCTCTCACGGGATGTGA

"Constitutive promoter" refers to a promoter that is capable of facilitating continuous transcription of a coding sequence or gene under its control and/or to which it is operably linked. Constitutive promoters and variants are well known in the art and include, but are not limited to, Ptac promoter, BBa_J23100, a constitutive Escherichia coli σS promoter (e.g., an osmY promoter (International Genetically Engineered Machine (iGEM) Registry of Standard Biological Parts Name BBa_J45992; BBa_J45993)), a constitutive Escherichia coli o32 promoter (e.g., htpG heat shock promoter (BBa_J45504)), a constitutive Escherichia coli σ70 promoter (e.g., lacq promoter (BBa_J54200; BBa_J56015), E. coli CreABCD phosphate sensing operon promoter (BBa_J64951), GlnRS promoter (BBa_K088007), lacZ promoter (BBa_KI 19000; BBa_KI 19001); M13K07 gene I promoter (BBa_M13101); M13K07 gene II promoter (BBa_M13102), M13K07 gene III promoter (BBa_M13103), M13K07 gene IV promoter (BBa_M13104), M13K07 gene V promoter (BBa_M13105), M13K07 gene VI promoter (BBa_M13106), M13K07 gene VIII promoter (BBa_M13108), M13110 (BBa_M13110)), a constitutive Bacillus subtilis oA promoter (e.g., promoter veg (BBa_K143013), promoter 43 (BBa_K143013), PliaG (BBa_K823000), PlepA (BBa_K823002), Pveg (BBa_K823003)), a constitutive Bacillus subtilis oB promoter (e.g., promoter etc (BBa_K143010), promoter gsiB (BBa_K143011)), a Salmonella promoter (e.g., Pspv2 from Salmonella (BBa_KI 12706), Pspv from Salmonella (BBa_KI 12707)), a bacteriophage T7 promoter (e.g., T7 promoter (BBa_I712074; BBa_I719005; BBa_J34814; BBa_J64997; BBa_K113010; BBa_K113011; BBa_K113012; BBa_R0085; BBa_R0180; BBa_R0181; BBa_R0182; BBa_R0183; BBa_Z0251; BBa_Z0252; BBa_Z0253)), and a bacteriophage SP6 promoter (e.g., SP6 promoter (BBa_J64998)).

An "inducible promoter" refers to a regulatory region that is operably linked to one or more genes, wherein expression of the gene(s) is increased in the presence of an inducer of said regulatory region. An "inducible promoter" refers to a promoter that initiates increased levels of transcription of the coding sequence or gene under its control in response to a stimulus or an exogenous environmental condition. A "directly inducible promoter" refers to a regulatory region, wherein the regulatory region is operably linked to a gene encoding a protein or polypeptide, where, in the presence of an inducer of said regulatory region, the protein or polypeptide is expressed. An "indirectly inducible promoter" refers to a regulatory system comprising two or more regulatory regions, for example, a first regulatory region that is operably linked to a first gene encoding a first protein, polypeptide, or factor, e.g., a transcriptional regulator, which is capable of regulating a second regulatory region that is operably linked to a second gene, the second regulatory region may be activated or repressed, thereby activating or repressing expression of the second gene. Both a directly inducible promoter and an indirectly inducible promoter are encompassed by "inducible promoter."

As used herein, the term "sequence identity" as used herein is understood as the relatedness between two amino acid sequences or between two nucleotide sequences and described by the degree of sequence identity or sequence complementarity. The sequence identity of a variant, homologue or orthologue as compared to a parent nucleotide or amino acid sequence indicates the degree of identity of two or more sequences. Two or more amino acid sequences may have the same or conserved amino acid residues at a corresponding position, to a certain degree, up to 100%. Two or more nucleotide sequences may have the same or conserved base pairs at a corresponding position, to a certain degree, up to 100%.

The term "expression construct" as used herein, means the vehicle, e.g. vectors or plasmids, by which a DNA sequence is introduced into a host cell so as to transform the host and promote expression (e.g. transcription and translation) of the introduced sequence. "Expression construct" as used herein includes both, autonomously replicating nucleotide sequences as well as genome integrating nucleotide sequences.

The terms "vector", "DNA vector" and "expression vector" mean the vehicle by which a DNA sequence e.g. a foreign gene) can be introduced into a host cell, so as to transform the host and promote expression (e.g. transcription and translation) of the introduced sequence. "Vector" as used herein includes both, autonomously replicating nucleotide sequences as well as genome integrating nucleotide sequences, such as artificial chromosomes. A common type of vector is a "plasmid", which generally is a self-contained molecule of double-stranded DNA that can readily accept additional (foreign) DNA and which can readily be introduced into a suitable host cell. Specifically, the term "vector" or "plasmid" refers to a vehicle by which a DNA or RNA sequence (e.g. a foreign gene) can be introduced into a host cell, so as to transform the host and promote expression (e.g. transcription and translation) of the introduced sequence.

As used herein, the term "transform" or "transformation" refers to the transfer of a nucleic acid fragment into a host bacterial cell, resulting in genetically-stable inheritance. Host bacterial cells comprising the transformed nucleic acid fragment are referred to as "recombinant" or "transgenic" or "transformed" organisms. The term "ROS reduction" or "reduction of ROS" refers to a decrease of any intracellular or extracellular ROS levels to levels similar ($\pm$ 20%) to those of cells not treated with an oxidising agent, including (but not limited to) UV or paraquat.

In some embodiments of the first aspect of the invention, the secretion signal is of SEQ ID No. 1 or a functional variant thereof that has at least 80%, 85%, 90%, 91%, 92%, 93%, 94%, 95%, 96%, 97%, 98%, or 99% sequence identity with the full/length sequence of SEQ ID NO 1 with the proviso that said functional variant can be processed by *C. acnes* cells to secrete the heterologous protein.

PPA_RS09745signal peptide (nt) (SEQ ID NO 4):
atgttcgtccaaatcgctgccagcctggcagccgcatcgtccattgcactcggcataccaggagctgcc

PPA_RS09745 signal peptide (aa) (SEQ ID NO 1):
MFVQIAASLAAASSIALGIPGAA

In some embodiments of the first aspect of the invention, optionally in combination with any of the previous or subsequent embodiments of the first aspect of the invention, the superoxide dismutase enzyme is the SodC-F1 from the enterohemorrhagic *E. coli* O157:H7 of SEQ ID NO 2 or any sequence that has at least 80%, 90%, 91%, 92%, 93%, 94%, 95%, 96%, 97%, 98%, or 99% identity with the full/length sequence of of SEQ ID No. 2 that when secreted in one or multiple copies by using the secretion signal of SEQ ID NO 1 is capable of reducing ROS levels.

SodC-F1 from E. coli O157:H7 (codon-optimized for C *acnes)*

SodC-F1 (nt) (SEQ ID NO 9):

(ATG)GAGCAGGAAGTGCCGATGAACCTGGTCTCGGCTGATGGCAAGGAGGTTTCC
ATCGGGAAGATCACCATCCAGGAGACCCCATACGGTCTGTTGTTCACCCCCGCCC
TGCACTCGCTGAGCGAGGGTATTCACGGTTTCCACGTCCACGAGAAGGGCAACTG
CGCCCCGGCGCTTAAAGACGGCAAGCCGGTCGCCGCGCTGTCCGCCGGTGGTCA
CTTTGACCCGAAGAATACTGGCAAGCACCTGGGCCCGTGGTCCCCTGACGGCCAC
cTcGGtGACCTGCCGGCCCTGTTCGTCACCCATGACGGCAAAGCCAACTACCCGGT
CCTGGCCCCGCGCCTCAACTCCCTGAAGGAGATCAAGGGCCGCTCcCTcATGCTcC
ACGCtGGtGGCGACAATCACCACGACCACCCAGAGCCCCTGGGCGGTGGCGGtGC
CCGCATGGCCTGCGGCATTATCCAGCATCATCACCATCACCACTGAtaa

SodC-F1 (aa) (SEQ ID NO 2):

(M)EQEVPMNLVSADGKEVSIGKITIQETPYGLLFTPALHSLSEGIHGFHVHEKGNCAPA
LKDGKPVAALSAGGHFDPKNTGKHLGPWSPDGHLGDLPALFVTHDGKANYPVLAPRL
NSLKEIKGRSLMLHAGGDNHHDHPEPLGGGGARMACGIIQ

It is noted that the SodC-F1 does not have a start codon (ATG) because it is fused to a N-terminal signal peptide, and the signal peptide does have the start codon.

In some embodiments of the first aspect of the invention, optionally in combination with any of the previous or subsequent embodiments of the first aspect of the invention, the superoxide dismutase enzyme is the SodC-F1 from the enterohemorrhagic *E. coli* O157:H7 of SEQ ID NO 2 and the secretion signal is of SEQ ID No. 1.

In some embodiments of the first aspect of the invention, optionally in combination with any of the previous or subsequent embodiments of the first aspect of the invention, the one or more transcription promoter sequences is the promoter of SEQ ID NO 3, 5 6, or 15 or any sequence that has at least 80%, 90%, 91%, 92%, 93%, 94%, 95%, 96%, 97%, 98%, or 99% identity with the full/length sequence of SEQ ID No. 3, 5, 6, or 15 that when operably linked to the heterologous protein is capable of expressing the said protein. In some embodiments of the first aspect of the invention, optionally in combination with any of the previous or subsequent embodiments of the first aspect of the invention, the one or more transcription promoter sequences is selected from the list consisting of: HspR C. acnes (PPA_RS10230), HspR Operon promoter temperature sensor from C. acnes (P(PPA_RS10245), OxyR C. acnes promoter ROS Sensing 1 (P(PPA_RS10005)), OxyR C. acnes promoter ROS sensing 2 (P(PPA_RS10010)), or OxyR C. acnes transcription factor (PPA_RS10015), or any sequence that has at least 80%, 90%, 91%, 92%, 93%, 94%, 95%, 96%, 97%, 98%, or 99% identity with the full/length sequence of any of these promoters.

pEND-100

Promoter (called "P (PPA_RS09745)") of SEQ ID NO 3:

acaccgacgatctgcacgggcgagtctaggggttatcggctacatgcgcgtcgccgcatcaggttcacgaaaccgaaa
ggcagacccaccccctaccgggcataccgttgccgccgacacttcgatgacactatgcagtgctacacttcaactgaaaa
tctatatgattttcgtcaattc

pEND-273

Promoter (called "P (PPA_RS09225)") of SEQ ID NO 5:

ggttgatctgtagtgctgccgggggccgtgggttccggcagcactgtggactacgggcgggacgcgtgccctctgtgtgagtt

actgccggtgccctttagctcctttcaatttggggagacgacgttgagaaggtatcgttaactgctggcttcgtgtgtgcggag

ccacaaccacgcccgcaggggggtgtggg

pEND-274

Promoter (called "P (PPA_RS07095)") of SEQ ID NO 6:

CCTGTCCTGATCATGATCCGTCCGGGGGCCGCTAGTTGGGCGGTCCCCGGACGA

TTTTGTGTACCCGCAGATGCGTGCCAGACAAGCGCCTCGGCTGTTTTGTGGGTTT

GTGTTGGTGGTGGGGGTGTGTGTAGTGTGTGTTGGGCCGTTGGGGGCTGCGTGT

GTGGTTCTTGGTGGTGGCTGTGTTTGGGATG

pEND-387

Promoter (called "BBa_J23119-TetO2", i.e. the aTC-inducible promoter) of SEQ ID NO 15:

CAGCCTGCGGTCCGGTTGACACCCTATCAGTGATAGAGTATAATGCTAGCTCGCT

GGGACGCCCG

In some embodiments of the first aspect of the invention, optionally in combination with any of the previous or subsequent embodiments of the first aspect of the invention, the one or more transcription promoter sequences is the synthetic E. *coli* consensus promoter of SEQ ID NO 3.

In some embodiments of the first aspect of the invention, optionally in combination with any of the previous or subsequent embodiments of the first aspect of the invention, the expression construct further comprises a ribosome binding sites (RBS): preferably the RBS is the *E. coli* RBS sequences of SEQ ID No 7, or 8 or any sequence that has at least 80%, 90%, 91%, 92%, 93%, 94%, 95%, 96%, 97%, 98%, or 99% identity with the full/length sequence of SEQ ID No. 7 or 8.

RBS (called "RBS_1") of SEQ ID NO 7:
TC CACTTATAAATATAGAG GAG GTTT GAT

RBS (called "RBS_2") of SEQ ID NO 8:
TAATTTTGTTTAACTTTAAGAAGGAGATATACAT

In some embodiments of the first aspect of the invention, optionally in combination with any of the previous or subsequent embodiments of the first aspect of the invention, the expression construct further comprises a ribosome binding sites (RBS): preferably the RBS is the *E. coli* RBS sequences SEQ ID No 7.

In some embodiments of the first aspect of the invention, optionally in combination with any of the previous or subsequent embodiments of the first aspect of the invention, the expression construct is of SEQ ID NO 9, 10, 11, or 16 or any sequence that has at least 80%, 90%, 91%, 92%, 93%, 94%, 95%, 96%, 97%, 98%, or 99% identity with the full/length sequence of SEQ ID No. 9, 10, 11, or 16.

pEND-100 of SEQ ID NO 9:

CAGCCTGCGGTCCGGacaccgacgatctgcacgggcgagtctaggggttatcggctacatgcgcgtcgccgc
atcaggttcacgaaaccgaaaggcagacccacccctaccgggcataccgttgccgccgacacttcgatgacactatgc
agtgctacacttcaactgaaaatctatatgattttcgtcaattcTCCACTTATAAATATAGAGGAGGTTTGA
TatgttcgtccaaatcgctgccagcctggcagccgcatcgtccattgcactcggcataccaggagctgccGAGCAGG
AAGTGCCGATGAACCTGGTCTCGGCTGATGGCAAGGAGGTTTCCATCGGGAAGAT
CACCATCCAGGAGACCCCATACGGTCTGTTGTTCACCCCCGCCCTGCACTCGCTG
AGCGAGGGTATTCACGGTTTCCACGTCCACGAGAAGGGCAACTGCGCCCCGGCG
CTTAAAGACGGCAAGCCGGTCGCCGCGCTGTCCGCCGGTGGTCACTTTGACCCG

AAGAATACTGGCAAGCACCTGGGCCCGTGGTCCCCTGACGGCCACcTcGGtGACCT
GCCGGCCCTGTTCGTCACCCATGACGGCAAAGCCAACTACCCGGTCCTGGCCCC
GCGCCTCAACTCCCTGAAGGAGATCAAGGGCCGCTCcCTcATGCTcCACGCtGGtG
GCGACAATCACCACGACCACCCAGAGCCCCTGGGCGGTGGCGGtGCCCGCATGG
CCTGCGGCATTATCCAGCATCATCACCATCACCACTGAtaaTCGCTGGGACGCCCG

pEND-273 of SEQ ID NO 10:

CAGCCTGCGGTCCGGggttgatctgtagtgctgccgggggccgtgggttccggcagcactgtggactacgggcg
ggacgcgtgccctctgtgtgagttactgccggtgccctttagctcctttcaatttggggagacgacgttgagaaggtatcgtta
actgctggcttcgtgtgtgcggagccacaaccacgcccgcagggggtgtgggTCGCTGGGACGCCCGTAG
AACTCTGTGAGGATAAAGTCTCCCTTACAGCCTGCGGTCCGGTAATTTTGTTTAACT
TTAAGAAGGAGATATACATatgttcgtccaaatcgctgccagcctggcagccgcatcgtccattgcactcggc
ataccaggagctgccGAGCAGGAAGTGCCGATGAACCTGGTCTCGGCTGATGGCAAGGA
GGTTTCCATCGGGAAGATCACCATCCAGGAGACCCCATACGGTCTGTTGTTCACC
CCCGCCCTGCACTCGCTGAGCGAGGGTATTCACGGTTTCCACGTCCACGAGAAGG
GCAACTGCGCCCCGGCGCTTAAAGACGGCAAGCCGGTCGCCGCGCTGTCCGCCG
GTGGTCACTTTGACCCGAAGAATACTGGCAAGCACCTGGGCCCGTGGTCCCCTGA
CGGCCACcTcGGtGACCTGCCGGCCCTGTTCGTCACCCATGACGGCAAAGCCAACT
ACCCGGTCCTGGCCCCGCGCCTCAACTCCCTGAAGGAGATCAAGGGCCGCTCcCT
cATGCTcCACGCtGGtGGCGACAATCACCACGACCACCCAGAGCCCCTGGGCGGTG
GCGGtGCCCGCATGGCCTGCGGCATTATCCAGCATCATCACCATCACCACTGAtaaT
CGCTGGGACGCCCG

pEND-274 of SEQ ID NO 11:

CAGCCTGCGGTCCGGCCTGTCCTGATCATGATCCGTCCGGGGGCCGCTAGTTGG
GCGGTCCCCGGACGATTTTGTGTACCCGCAGATGCGTGCCAGACAAGCGCCTCG
GCTGTTTTGTGGGTTTGTGTTGGTGGTGGGGGTGTGTGTAGTGTGTGTTGGGCCG
TTGGGGGCTGCGTGTGTGGTTCTTGGTGGTGGCTGTGTTTGGGATGTCGCTGGGA
CGCCCGTAGAACTCTGTGAGGATAAAGTCTCCCTTACAGCCTGCGGTCCGGTAATT

TTGTTTAACTTTAAGAAGGAGATATACATatgttcgtccaaatcgctgccagcctggcagccgcatcgtc
cattgcactcggcataccaggagctgccGAGCAGGAAGTGCCGATGAACCTGGTCTCGGCTGA
TGGCAAGGAGGTTTCCATCGGGAAGATCACCATCCAGGAGACCCCATACGGTCTG
TTGTTCACCCCCGCCCTGCACTCGCTGAGCGAGGGTATTCACGGTTTCCACGTCC
ACGAGAAGGGCAACTGCGCCCCGGCGCTTAAAGACGGCAAGCCGGTCGCCGCGC
TGTCCGCCGGTGGTCACTTTGACCCGAAGAATACTGGCAAGCACCTGGGCCCGTG
GTCCCCTGACGGCCACcTcGGtGACCTGCCGGCCCTGTTCGTCACCCATGACGGCA
AAGCCAACTACCCGGTCCTGGCCCCGCGCCTCAACTCCCTGAAGGAGATCAAGG
GCCGCTCcCTcATGCTcCACGCtGGtGGCGACAATCACCACGACCACCCAGAGCCC
CTGGGCGGTGGCGGtGCCCGCATGGCCTGCGGCATTATCCAGCATCATCACCATC
ACCACTGAtaaTCGCTGGGACGCCCG

pEND-387 of SEQ ID NO 16:

CAGCCTGCGGTCCGGTTGACACCCTATCAGTGATAGAGTATAATGCTAGCTCGCT
GGGACGCCCGTAGAACTCTGTGAGGATAAAGTCTCCCTTACAGCCTGCGGTCCGG
TAATTTTGTTTAACTTTAAGAAGGAGATATACATatgttcgtccaaatcgctgccagcctggcagccg
catcgtccattgcactcggcataccaggagctgccGAGCAGGAAGTGCCGATGAACCTGGTCTCGG
CTGATGGCAAGGAGGTTTCCATCGGGAAGATCACCATCCAGGAGACCCCATACGG
TCTGTTGTTCACCCCCGCCCTGCACTCGCTGAGCGAGGGTATTCACGGTTTCCAC
GTCCACGAGAAGGGCAACTGCGCCCCGGCGCTTAAAGACGGCAAGCCGGTCGCC
GCGCTGTCCGCCGGTGGTCACTTTGACCCGAAGAATACTGGCAAGCACCTGGGC
CCGTGGTCCCCTGACGGCCACcTcGGtGACCTGCCGGCCCTGTTCGTCACCCATGA
CGGCAAAGCCAACTACCCGGTCCTGGCCCCGCGCCTCAACTCCCTGAAGGAGAT
CAAGGGCCGCTCcCTcATGCTcCACGCtGGtGGCGACAATCACCACGACCACCCAG
AGCCCCTGGGCGGTGGCGGtGCCCGCATGGCCTGCGGCATTATCCAGCATCATCA
CCATCACCACTGAtaaTCGCTGGGACGCCCGCTCGAGCAATAAACAGTTGATAGGG
CTTCTCCGTTACCATGGTTCAGCCAAAAACTTAAGACCGCCGGTCTTGTCCACTA
CCTTGCAGTAATGCGGTGGACAGGATCGGCGGTTTTCTTTTCTCTTCTCAATTCTTC
TGACCTGTAACGAATAATAGATAGTAAAGTAGTCTCCGATTGAGTTTTCTCTGCCGA
GTCCCACCCAGTTCTGTGATTTCAGTAAGTTGGTAATTGATACACTGTTGCGAGAA

CTGgTGCCTGGTAGTAGATAGGTTGTTATTGAGTAAGAAGGTAAAGTGAACGAAAT
CCCTGAAACTGAGACTGTAGAAATAAGCTTGTCCAGACTATTGGATCCAAGAGAT
TTCTACACGATTGAGCACTGTCTCCTGCAGGCTCGGTACCAAATTCCAGAAAAGAG
GCCTCCCGAAAGGGGGGCCTTTTTTCGTTTTGGTCCTAATAGATAAAGGATAGGTC
TGGTAGTGTTGTTCGTTCTCGCAGGTAAATCAATAATACTCtGCAGTTCCGTAGACT
TTTCAGTGGGACAGGGTAGCGATAACAGATAGATTGTAATAAGACACAGTAGGTGC
TCGTAGTTGCGTGAAGAGAACCGCTCAGGAAATCCAGTCAGAAGTATTGGTAATCG
TTGAAAACTCAGTCGACCAGCCTGCGGTCCGGacaccgacgatctgcacgggcgagtctaggg g
ttatcggctacatgcgcgtcgccgcatcaggttcacgaaaccgaaaggcagacccaccccctaccgggcataccgttgc
cgccgacacttcgatgacactatgcagtgctacacttcaactgaaaatctatatgattttcgtcaattcTCGCTGGGAC
GCCCGTAGAACTCTGTGAGGATAAAGTCTCCCTTACAGCCTGCGGTCCGGTAATTT
TGTTTAACTTTAAGAAGGAGATATACATATGTCAAGACTAGATAAGAGCAAGGTCAT
TAACAGCGCACTGGAGTTACTAAACGAAGTTGGAATAGAGGGATTGACTACACGAA
AATTAGCACAGAAATTAGGAGTAGAACAACCGACACTTTATTGGCACGTTAAAAATA
AAAGAGCTTTGCTGGATGCTTTAGCTATAGAAATGCTAGACCGACACCACACACAT
TTTTGCCCCTTAGAAGGAGAAAGTTGGCAAGATTTCCTACGTAACAACGCCAAATC
ATTTAGATGCGCGCTATTATCACACCGCGACGGCGCCAAAGTTCACCTCGGGACA
AGACCTACGGAGAAGCAATACGAAACCCTAGAGAATCAATTAGCCTTTCTATGTCA
ACAAGGATTCAGTTTGGAAAACGCCCTCTACGCCCTATCAGCCGTTGGTCATTTTA
CATTGGGATGCGTTTTAGAGGATCAGGAACATCAAGTAGCAAAAGAAGAAAGAGA
GACACCCACCACCGACAGCATGCCACCATTATTACGTCAAGCTATTGAACTATTCG
ATCATCAGGGAGCGGAACCGGCATTTTTATTTGGCCTCGAATTAATCATATGTGGT
TTAGAAAACAATTAAAGTGTGAATCTGGATCCCATCATCACCATCACCACTGAtaaT
CGCTGGGACGCCCG

In some embodiments of the first aspect of the invention, optionally in combination with any of the previous or subsequent embodiments of the first aspect of the invention, the expression construct is comprised in a plasmid, preferably a plasmid capable of replicating in *C. acnes* and comprising an origin of replication for *C. acnes.*

In some embodiments of the first aspect of the invention, optionally in combination with any of the previous or subsequent embodiments of the first aspect of the invention, the strains are mutant live auxotrophic *Cutibacterium acnes (C. acnes)* bacterial strains, preferably for Lysine, Histidine or Proline, with the proviso that said mutant live auxotrophic *C. acnes* bacterial strains do not comprise any heterologous antibiotic resistance genes.

In some embodiments of the first aspect of the invention, optionally in combination with any of the previous or subsequent embodiments of the first aspect of the invention, the expression construct is comprised in a plasmid and the plasmid comprising the said expression construct is selected from any one of SEQ ID NO 12, 13, 14, or 17 or any sequence that has at least 80%, 90%, 91%, 92%, 93%, 94%, 95%, 96%, 97%, 98%, or 99% identity with the full/length sequence of SEQ ID No. 12, 13 14, or 17.

pEND-100 of SEQ ID NO 12:

GTGCGTGCGGGCGTGAGCGTTTCTACGCTGCGGCGCAGGAAATCAGAGCTTGAG
GCTGCCGGAGCGACGGTAGACCCGTCCGGTTGGGTGGTGCCACTGCGTGCACTC
AAGGTCGTTTTTGGGGTGTCAGATGAGACCTCGAATGCGAATACCAGTAAATCCTG
ACCTGTGAGTTGTGAATTCATAGGATAGATTCTGGAAACTTTACCGTCCGAGCTCC
AGCCTGCGGTCCGGacaccgacgatctgcacgggcgagtctaggggttatcggctacatgcgcgtcgccgcat
caggttcacgaaaccgaaaggcagacccaccccctaccgggcataccgttgccgccgacacttcgatgacactatgca
gtgctacacttcaactgaaaatctatatgattttcgtcaattcTCCACTTATAAATATAGAGGAGGTTTGAT
atgttcgtccaaatcgctgccagcctggcagccgcatcgtccattgcactcggcataccaggagctgccGAGCAGGA
AGTGCCGATGAACCTGGTCTCGGCTGATGGCAAGGAGGTTTCCATCGGGAAGATC
ACCATCCAGGAGACCCCATACGGTCTGTTGTTCACCCCCGCCCTGCACTCGCTGA
GCGAGGGTATTCACGGTTTCCACGTCCACGAGAAGGGCAACTGCGCCCCGGCGC
TTAAAGACGGCAAGCCGGTCGCCGCGCTGTCCGCCGGTGGTCACTTTGACCCGA
AGAATACTGGCAAGCACCTGGGCCCGTGGTCCCCTGACGGCCACcTcGGtGACCT
GCCGGCCCTGTTCGTCACCCATGACGGCAAAGCCAACTACCCGGTCCTGGCCCC
GCGCCTCAACTCCCTGAAGGAGATCAAGGGCCGCTCcCTcATGCTcCACGCtGGtG

GCGACAATCACCACGACCACCCAGAGCCCCTGGGCGGTGGCGGtGCCCGCATGG
CCTGCGGCATTATCCAGCATCATCACCATCACCACTGAtaaTCGCTGGGACGCCCG
CTCGAGCAATAAACAGTTGATAGGGCTTCTCCGTTACCATGGTTCAGCCAAAAAC
TTAAGACCGCCGGTCTTGTCCACTACCTTGCAGTAATGCGGTGGACAGGATCGGC
GGTTTTCTTTTCTCTTCTCAAGCTAGCGAAATCCCTGAAACTGAGACTGTAGAAAT
AAGCTTCCGAAACTGAACCTGTTATTTTACGCTACGGAcgCCCGGTCATGACGCTG
AGTTAGTGGCGCAGCTGCGCTCTGAGAACGAGTTTTTACGGCGTCAGGTCGAGCA
GCAGGCGCGCACGATCGAACGGCAGGCTGAGGCACACGCGGTGGTCTCAGCGC
AGCTCACACGGGTTGGCCAGCTTGAGGCCGGCGACGCAGCAGCACCGACACTGG
CACCCGTTGAAAGGCCGGCTCCGCGACGGCGGTGGTGGCAGCGTCGGTAGCGG
TCAGGATCGCTCTGGCGTGACGAGTGTGTCTGGCAGTGCGAACAGTTGCTCGACC
AGTGGCAGCAGAAGCGAGATCGCTGCGTGGTGCTGTTCCTCGGTCAGTTCGTCGA
GGACTGGCGGGTCTTGCTGCGTCCAGCCGATCGCCTCGGCGGCCAAGGTCAGTT
CCAAGCTGTGCCAACGCACACGCCCCTCGGCTGACAGCTGAGTCTCGAACTGTGC
AACTGGACCGGCCGGAAGATGCACGTTGCCGAGGTCGTGAGTGGCCAAGCGCAC
GTCAAAGAGTGCTGCTTCGTAGCCGCGCAGAAATGGCAGTGCTCGGTCGATTCGG
ATCGGCCTGCCCAGGTACATTCCGGGCCGCTTGATGAACGCCTCCGCGTAGAAGC
GCACCGTTCTCGGCCCGGCCTCGTGATCTGTCACTGTGCACGCTCCTCTCGATGG
TTCTCGACGCTACCGGAGACCACCGACGTTCATGCCCAGCGCAGCGACCTGAAAG
GACCAAGCCGAGTTAGCCGTGCTAACCGTATAGCTTGCTCCGTCGCCTCTGAGGG
CAACCACCTGCGCAGCAGGTGGGCGGCAGCCCGCGCGCAAGCGCCTACCGGGT
TTGGGCACAGCCCATAAATCAACGCCTCCGGTGTTGAAGCGATCGTGTGTCACGA
TTGCTATGCTTGCTACCCCTTCAGGGTTTTCGTATACACAAATCAAGTTTTTTCGTA
TACGCTAATGCCATGAGTGAGCATCTACTGCACGGCAAGCCCGTCACCAACGAGC
AGATTCAGGCATGGGCAGACGAGGCCGAGGCCGGATACGACCTGCCCAAACTCC
CCAAGCCACGGCGCGGACGCCCGCCCGTAGGAGACGGTCCGGGCACCGTCGTA
CCCGTGCGTCTCGACGCGGCCACCGTTGCCGCTCTCACAGAACGAGCAACAGCC
GAGGGCATCACGAACCGTTCAGACGCGATCCGAGCCGCAGTCCACGAGTGGACA
CGGGTTGCCTGACCTCCACGACTCAGCACGCAAGCACTACCAACGAGACCGGCTC
GACGACACGGCCGTGCTCTACGCGGCCACCCACGTTCTCAACTCCCGGCCACTC
GACGACGAAGACGACCCGCGCCGCTGGCTCATGATCGGAACCGACCCAGCAGGC

CGCCTACTCGAACTCGTCGCACTGATCTACGACGACGGCTACGAACTGATCATCC
ACGCAATGAAAGCCCGCACCCAATACCTCGACCAGCTCTAACCAAGAAAGGAACC
TGATGAGCGACCAGCTAGACAGCGACCGCAACTACGACCCGATGATCTTCGACGT
GATGCGCGAGACCGCGAACCGCGTCGTCGCCACGTACGTTGCATGGGAAGATGA
AGCCGCTGATCCCCGCGAGGCTGCGCACTGGCAGGCCGAGCGATTCCGCACCCG
GCACGAGGTGCGCGCCGTCGACCCTGACAGCCGGCGAGCAGTTCAGGCGAAGAT
CGCACAGCTGCGCGAGGAACTAGCCGCAATGCCCGAACACGCCCCAGCCATCCC
TTGGAGCAGGTGGCAGCGTCAGGGGAGTCGGGGGATGTTTGGCAGGGGATGTGG
AAAGAGAGTTCGCTTTGCTCACATGGCTCAACCGGGTAACTAACTGATATGGGGTC
TTCGTCGCCCACTTTGAACACGCCGAGGAATGGACCACGCTGAACGTGACTCGCA
TGCTTCACTGCATGTATGGATTCGTTCGAGACGTTGTTCCCTGAGAGCTGGCTGCC
ACGCAAGCCGCTGGCGTCAGCCGAGAAGTCTGGGGCGTACCGGCACGTGACTCG
GCAGAGGGCGCTGGAGCTGCCTTACATCGAAGCGAACCCGTTGGTCATGCAGTC
CTTGGTCATCACCGATCGAGATGCTTCGGATGCTGACTGGGCCGCAGACCTCGCT
GGGCTGCCTTCACCGTCCTACGTGTCCATGAACCGTGTCACGACCACCGGACACA
TCGTCTATGCCTTGAAGAACCCTGTGTGTCTGACCGATGCCGCGCGGCGACGGCC
TATCAACCTGCTCGCCCGCGTCGAGCAGGGCCTATGCGACGTTCTCGGCGGCGAT
GCATCCTACGGGCACCGGATCACAAAGAACCCGCTCAGCACCGCCCATGCGACC
CTCTGGGGCCCCGCAGACGCGCTCTACGAGCTGCGCGCCCTCGCACACACCCTC
GACGAGATCCACGCACTGCCGGAGGCAGGGAACCCGCGTCGCAACGTCACCCGA
TCAACGGTCGGCCGCAACGTCACCCTGTTCGACACCACCCGCATGTGGGCATACC
GGGCCGTCCGGCACTCCTGGGGCGGCCCGGTCGCCGAATGGGAGCACACCGTA
TTCGAGCACATCCACCTACTGAACGAGACGATCATCGCCGACGAATTCGCCACAG
GCCCCCTCGGCTTGAACGAACTTAAGCACTTATCTCGATCCATTTCCCGATGGGTC
TGGCGCAACTTCACCCCCGAAACCTTCCGCGCACGCCAGAAAGCGATCAGCCTCC
GTGGAGCATCCAAAGGCGGCAAAGAAGGCGGCCACAAAGGCGGCATTGCCAGTG
GCGCATCACGGCGCGCCCATACCCGTCAACAGTTCTTGGAGGGTCTCTCATGACC
ACACGTGAACGTCTCCCCCGCAACGGCTACAGCATCGCCGCTGCTGCGAAAAAGC
TCGGTGTCTCCGAGTCCACCGTCAAGCGGTGGACTTCCGAGCCACGCGAGGAGTT
CGTGGCCCGCGTTGCCGCACGCCACGCGCGGATTCGTGAGCTgCGCTCGGAGGG
TCAGAGCATGCGTGCGATTGCTGCCGAGGTCGGGGTTTCCGTGGGCACCGTGCA

CTACGCGCTGAACAAGAATCGAACTGACGCATGACCGTAACGCCGCACGATGAGC
ATTTTCTTGATCGTGCACCGCTTGGCACTACGTTCGCGTGCGGTTGCACAGTGCG
CGCCACGTTCTTATCCTGCGGCCATTGTGGCTACAGCCAATGGGGGGCATCAGCA
ACGGACGTTGAACCCGGTGGGCAAGTGTTACTCAGGGGGACATGCCCAGTCTGC
GGCGCTCGGATTGACGGTATGGCAGTCGTGCATGCGGCCCCACCGTCAAACTCAT
TCAGGTATCAGTGAGAACCCTCATGGCACCCCTCGTGACACGTTCTCGTTGCGAT
CAGCTGCGGCCAAGCTTGATCTTCGGGCAGCGTTGGGTCCTGGCCACGGGTGCG
CATGATCGTGCTCCTGTCGTTGAGGACCCGGCTAGGCTGGCGGGGTTGCCTTACT
GGTTAGCAGAATGAATCACCGATACGCGAGCGAACGTGAAGCGACTGCTGCTGCA
AAACGTCTGCGACCTGAGCAACAACATGAATGGTCTTCGGTTTCCGTGTTTCGTAA
AGTCTGGAAACGCGGAAGTCAGCGCCCTGCACCATTATGTTCCGGATCTGCATCG
CAGGATGCTGCTGGCTACCCTGTGGAACACCTACATCTGTATTAACGAAGCGCTG
GCATTGACCCTGAGTGATTTTTCTCTGGTCCCGCCGCATCCATACCGCCAGTTGTT
TACCCTCACAACGTTCCAGTAACCGGGCATGTTCATCATCAGTAACCCGTATCGTG
AGCATCCTCTCGTTTCATCGGTATCATTACCCCCATGAACAGAAATCCCCCTTAC
ACGGAGGCATCAGTGACCAAACAGGAAAAAACCGCCCTTAACATGGCCCGCTTTA
TCAGAAGCCAGACATTAACGCTTCTGGAGAAACTCAACGAGCTGGACGCGGATGA
ACAGGCAGACATCTGTGAATCGCTTCACGACCACGCTGATGAGCTTTACCGCAGC
TGCCTCGCGCGTTTCGGTGATGACGGTGAAAACCTCTGACACATGCAGCTCCCGG
AGACGGTCACAGCTTGTCTGTAAGCGGATGCCGGGAGCAGACAAGCCCGTCAGG
GCGCGTCAGCGGGTGTTGGCGGGTGTCGGGGCGCAGCCATGACCCAGTCACGTA
GCGATAGCGGAGTGTATACTGGCTTAACTATGCGGCATCAGAGCAGATTGTACTG
AGAGTGCACCATATGCGGTGTGAAATACCGCACAGATGCGTAAGGAGAAAATACC
GCATCAGGCGCTCTTCCGCTTCCTCGCTCACTGACTCGCTGCGCTCGGTCGTTCG
GCTGCGGCGAGCGGTATCAGCTCACTCAAAGGCGGTAATACGGTTATCCACAGAA
TCAGGGGATAACGCAGGAAAGAACATGTGAGCAAAAGGCCAGCAAAAGGCCAGG
AACCGTAAAAAGGCCGCGTTGCTGGCGTTTTTCCATAGGCTCCGCCCCCCTGACG
AGCATCACAAAAATCGACGCTCAAGTCAGAGGTGGCGAAACCCGACAGGACTATA
AAGATACCAGGCGTTTCCCCCTGGAAGCTCCCTCGTGCGCTCTCCTGTTCCGACC
CTGCCGCTTACCGGATACCTGTCCGCCTTTCTCCCTTCGGGAAGCGTGGCGCTTT
CTCATAGCTCACGCTGTAGGTATCTCAGTTCGGTGTAGGTCGTTCGCTCCAAGCTG

GGCTGTGTGCACGAACCCCCCGTTCAGCCCGACCGCTGCGCCTTATCCGGTAACT
ATCGTCTTGAGTCCAACCCGGTAAGACACGACTTATCGCCACTGGCAGCAGCCAC
TGGTAACAGGATTAGCAGAGCGAGGTATGTAGGCGGTGCTACAGAGTTCTTGAAG
TGGTGGCCTAACTACGGCTACACTAGAAGGACAGTATTTGGTATCTGCGCTCTGCT
GAAGCCAGTTACCTTCGGAAAAGAGTTGGTAGCTCTTGATCCGGCAAACAAACCA
CCGCTGGTAGCGGTGGTTTTTTTGTTTGCAAGCAGCAGATTACGCGCAGAAAAAA
GGATCTCAAGAAGATCCTTTGATCTTTTCTACGGGGTCTGACGCTCAGTGGAACGA
AAACTCACGTTAAGGGATTTTGGTCATGAGATTATCAAAAAGGATCTTCACCTAGAT
CCTTTTAAATTAAAAATGAAGTTTTAAATCAATCTAAAGTATATATGAGTAAACTTGG
TCTGACAGTTACCAATGCTTAATCAGTGAGGCACCTATCTCAGCGATCTGTCTATTT
CGTTCATCCATAGTTGCCTGACTCCCCGTCGTGTAGATAACTACGATACGGGAGG
GCTTACCATCTGGCCCCAGTGCTGCAATGATACCGCGAGACCCACGCTCACCGGC
TCCAGATTTATCAGCAATAAACCAGCCAGCCGGAAGGGCCGAGCGCAGAAGTGGT
CCTGCAACTTTATCCGCCTCCATCCAGTCTATTAATTGTTGCCGGGAAGCTAGAGT
AAGTAGTTCGCCAGTTAATAGTTTGCGCAACGTTGTTGCCATTGCTGCAGGCATCG
TGGTGTCACGCTCGTCGTTTGGTATGGCTTCATTCAGCTCCGGTTCCCAACGATCA
AGGCGAGTTACATGATCCCCCATGTTGTGCAAAAAAGCGGTTAGCTCCTTCGGTCC
TCCGATCGTTGTCAGAAGTAAGTTGGCCGCAGTGTTATCACTCATGGTTATGGCAG
CACTGCATAATTCTCTTACTGTCATGCCATCCGTAAGATGCTTTTCTGTGACTGGTG
AGTACTCAACCAAGTCATTCTGAGAATAGTGTATGCGGCGACCGAGTTGCTCTTGC
CCGGCGTCAACACGGGATAATACCGCGCCACATAGCAGAACTTTAAAAGTGCTCA
TCATTGGAAAACGTTCTTCGGGGCGAAAACTCTCAAGGATCTTACCGCTGTTGAGA
TCCAGTTCGATGTAACCCACTCGTGCACCCAACTGATCTTCAGCATCTTTTACTTTC
ACCAGCGTTTCTGGGTGAGCAAAAACAGGAAGGCAAAATGCCGCAAAAAAGGGAA
TAAGGGCGACACGGAAATGTTGAATACTCATACTCTTCCTTTTTCAATATTATTGAA
GCATTTATCAGGGTTATTGTCTCATGAGCGGATACATATTTGAATGTATTTAGAAAA
ATAAACAAATAGGGGTTCCGCGCACATTTCCCCGAAAGTGCCACCTGACGTCTAA
GAAACCATTATTATCATGACATTAACCTATAAAAATAGGCGTATCACGAGGCCCTTT
CGTCTTCAAGAATTCAAGCTTGTCGACGTTAACCTGCAGGCATGCGGATCCGGTAC
CAGCTGTCTCTTATACACATCTCTGCAGTTAACGCTGACCCGGACCACCACGACGA
CCCGGACGACCACCGGTACGACCACCACCAGACGGACCGCGGCGACCACCCTGG

TCACCCTGTTCACGTTCACCGGTACGACCGTCGTCACGGTTACGACCAGAAGAAC
GACGGTCACCGTGACCAGACGCTTGCCGGTCTCTGCGATCTTGGCCCCCGGTAC
GTCTGTCACCACGGTCCTGACCACCGGTACGACGGTCGCCCCTATCCTGTCCACC
CGTCCTACGACCACGCTGCGGCTGACCACCACGCGGTTCGTTTTTCTGGTCCAGA
CGTTCGAACAGACGCAGCCACTGTTCCGGACGAACGTAAGCAACAACAGCACCAC
CACCAACACCAGCGTGGTCCAGAGCAGCTTCAACACGACGACGCGGGTATTTACG
CAGCAGAGAAGCCTGGATGTTACCACCAACACCGGTGAAGCACAGTTCAACCATA
GATTCGTAACGTTCCAGAGCAGCACCTTCCAGCAGCGGTTCAGCACGACGACGCA
GACGCATGATAGCAGAGTCAACTTTCGGAACCGGTTTGAACAGACGACGATCGAC
TTTTTCAACGAATTCCCATTCGAACAGCGGCCAGGTCATAACGGTCAGACGAGACC
AACGACCGTAGTCACCGGTACGTTTACGAGCGAATTCCAGCTGGGTAACCATGGT
AGCGGTTTCGATGGTCGGAGCTTCCAGGCACCAGTCAACGATAGCAGAGGTGATA
CCGTACGGGATAGCACCAACGAAAGCGAACGGTTCCGGCGGCGGTTCAGCGGTC
AGGAAGTCAGCGTTAACAACTTCGATGTTCGGGTGACCAGACAGTTTTTCACGCAG
AGATTTAGCCAGACGCGGGTCGATTTCGTAAGAGGTAACCTGACGAGCACGGTCA
GCCAGTTCACGGGTCAGCAGACCTTCACCCGGACCAGCTTCCAGAACCGGCAGG
TCCGGACGCAGTTCAGCGGTTTCAGCGATACGAGCGATGGTTTTACGGTCACGCA
GGAAGTTCTGGCCGAATTGGCGGCGGTTACGGTCACGCTCGGTACGGCCGAGGA
CGGGGCGATTCTGGTTGGGGTGCTGGCGGTCTTGGTTGCGGCGGCGGGGGCGG
GGCTGTTCATCGGAGGAGGACATGACGATCTCCCGATGTGTCGTACGTATGGGAA
TTGACGAAAATCATATAGATTTTCAGTTGAAGTGTAGCACTGCATAGTGTCATCGAA
GTGTCGGCGGCAACGGTATGCCCGGTAGGGGGTGGGTCTGCCTTTCGGTTTCGT
GAACCTGATGCGGCGACGCGCATGTAGCCGATAACCCCTAGACTCGCCCGTGCA
GATCGTCGGTGTGGATCCAGATGTGTATAAGAGACAGCTGGTACCGGCCGCTGCT

pEND-273 of SEQ ID NO 13:

GTGCGTGCGGGCGTGAGCGTTTCTACGCTGCGGCGCAGGAAATCAGAGCTTGAG
GCTGCCGGAGCGACGGTAGACCCGTCCGGTTGGGTGGTGCCACTGCGTGCACTC
AAGGTCGTTTTTGGGGTGTCAGATGAGACCTCGAATGCGAATACCAGTAAATCCTG
ACCTGTGAGTTGTGAATTCATAGGATAGATTCTGGAAACTTTACCGTCCGAGCTCC

AGCCTGCGGTCCGGggttgatctgtagtgctgccgggggccgtgggttccggcagcactgtggactacgggcgg
gacgcgtgccctctgtgtgagttactgccggtgccctttagctcctttcaatttggggagacgacgttgagaaggtatcgttaa
ctgctggcttcgtgtgtgcggagccacaaccacgcccgcaggggggtgtgggTCGCTGGGACGCCCGTAGA
ACTCTGTGAGGATAAAGTCTCCCTTACAGCCTGCGGTCCGGTAATTTTGTTTAACTT
TAAGAAGGAGATATACATatgttcgtccaaatcgctgccagcctggcagccgcatcgtccattgcactcggcat
accaggagctgccGAGCAGGAAGTGCCGATGAACCTGGTCTCGGCTGATGGCAAGGAG
GTTTCCATCGGGAAGATCACCATCCAGGAGACCCCATACGGTCTGTTGTTCACCCC
CGCCCTGCACTCGCTGAGCGAGGGTATTCACGGTTTCCACGTCCACGAGAAGGG
CAACTGCGCCCCGGCGCTTAAAGACGGCAAGCCGGTCGCCGCGCTGTCCGCCGG
TGGTCACTTTGACCCGAAGAATACTGGCAAGCACCTGGGCCCGTGGTCCCCTGAC
GGCCACcTcGGtGACCTGCCGGCCCTGTTCGTCACCCATGACGGCAAAGCCAACTA
CCCGGTCCTGGCCCCGCGCCTCAACTCCCTGAAGGAGATCAAGGGCCGCTCcCTc
ATGCTcCACGCtGGtGGCGACAATCACCACGACCACCCAGAGCCCCTGGGCGGTG
GCGGtGCCCGCATGGCCTGCGGCATTATCCAGCATCATCACCATCACCACTGAtaaT
CGCTGGGACGCCCGCTCGAGCAATAAACAGTTGATAGGGCTTCTCCGTTACCATG
GTTCAGCCAAAAAACTTAAGACCGCCGGTCTTGTCCACTACCTTGCAGTAATGCGG
TGGACAGGATCGGCGGTTTTCTTTTCTCTTCTCAAGCTAGCGAAATCCCTGAAACT
GAGACTGTAGAAAATAAGCTTCCGAAACTGAACCTGTTATTTTACGCTACGGAcgCC
CGGTCATGACGCTGAGTTAGTGGCGCAGCTGCGCTCTGAGAACGAGTTTTTACGG
CGTCAGGTCGAGCAGCAGGCGCGCACGATCGAACGGCAGGCTGAGGCACACGC
GGTGGTCTCAGCGCAGCTCACACGGGTTGGCCAGCTTGAGGCCGGCGACGCAGC
AGCACCGACACTGGCACCCGTTGAAAGGCCGGCTCCGCGACGGCGGTGGTGGCA
GCGTCGGTAGCGGTCAGGATCGCTCTGGCGTGACGAGTGTGTCTGGCAGTGCGA
ACAGTTGCTCGACCAGTGGCAGCAGAAGCGAGATCGCTGCGTGGTGCTGTTCCTC
GGTCAGTTCGTCGAGGACTGGCGGGTCTTGCTGCGTCCAGCCGATCGCCTCGGC
GGCCAAGGTCAGTTCCAAGCTGTGCCAACGCACACGCCCCTCGGCTGACAGCTG
AGTCTCGAACTGTGCAACTGGACCGGCCGGAAGATGCACGTTGCCGAGGTCGTG
AGTGGCCAAGCGCACGTCAAAGAGTGCTGCTTCGTAGCCGCGCAGAAATGGCAGT
GCTCGGTCGATTCGGATCGGCCTGCCCAGGTACATTCCGGGCCGCTTGATGAACG
CCTCCGCGTAGAAGCGCACCGTTCTCGGCCCGGCCTCGTGATCTGTCACTGTGCA
CGCTCCTCGATGGTTCTCGACGCTACCGGAGACCACCGACGTTCATGCCCAGC

GCAGCGACCTGAAAGGACCAAGCCGAGTTAGCCGTGCTAACCGTATAGCTTGCTC
CGTCGCCTCTGAGGGCAACCACCTGCGCAGCAGGTGGGCGGCAGCCCGCGCGC
AAGCGCCTACCGGGTTTGGGCACAGCCCATAAATCAACGCCTCCGGTGTTGAAGC
GATCGTGTGTCACGATTGCTATGCTTGCTACCCCTTCAGGGTTTTCGTATACACAA
ATCAAGTTTTTTCGTATACGCTAATGCCATGAGTGAGCATCTACTGCACGGCAAGC
CCGTCACCAACGAGCAGATTCAGGCATGGGCAGACGAGGCCGAGGCCGGATACG
ACCTGCCCAAACTCCCCAAGCCACGGCGCGGACGCCCGCCCGTAGGAGACGGTC
CGGGCACCGTCGTACCCGTGCGTCTCGACGCGGCCACCGTTGCCGCTCTCACAG
AACGAGCAACAGCCGAGGGCATCACGAACCGTTCAGACGCGATCCGAGCCGCAG
TCCACGAGTGGACACGGGTTGCCTGACCTCCACGACTCAGCACGCAAGCACTACC
AACGAGACCGGCTCGACGACACGGCCGTGCTCTACGCGGCCACCCACGTTCTCA
ACTCCCGGCCACTCGACGACGAAGACGACCCGCGCCGCTGGCTCATGATCGGAA
CCGACCCAGCAGGCCGCCTACTCGAACTCGTCGCACTGATCTACGACGACGGCTA
CGAACTGATCATCCACGCAATGAAAGCCCGCACCCAATACCTCGACCAGCTCTAA
CCAAGAAAGGAACCTGATGAGCGACCAGCTAGACAGCGACCGCAACTACGACCC
GATGATCTTCGACGTGATGCGCGAGACCGCGAACCGCGTCGTCGCCACGTACGTT
GCATGGGAAGATGAAGCCGCTGATCCCCGCGAGGCTGCGCACTGGCAGGCCGAG
CGATTCCGCACCCGGCACGAGGTGCGCGCCGTCGACCCTGACAGCCGGCGAGCA
GTTCAGGCGAAGATCGCACAGCTGCGCGAGGAACTAGCCGCAATGCCCGAACAC
GCCCCAGCCATCCCTTGGAGCAGGTGGCAGCGTCAGGGGAGTCGGGGGATGTTT
GGCAGGGGATGTGGAAAGAGAGTTCGCTTTGCTCACATGGCTCAACCGGGTAACT
AACTGATATGGGGTCTTCGTCGCCCACTTTGAACACGCCGAGGAATGGACCACGC
TGAACGTGACTCGCATGCTTCACTGCATGTATGGATTCGTTCGAGACGTTGTTCCC
TGAGAGCTGGCTGCCACGCAAGCCGCTGGCGTCAGCCGAGAAGTCTGGGGCGTA
CCGGCACGTGACTCGGCAGAGGGCGCTGGAGCTGCCTTACATCGAAGCGAACCC
GTTGGTCATGCAGTCCTTGGTCATCACCGATCGAGATGCTTCGGATGCTGACTGG
GCCGCAGACCTCGCTGGGCTGCCTTCACCGTCCTACGTGTCCATGAACCGTGTCA
CGACCACCGGACACATCGTCTATGCCTTGAAGAACCCTGTGTGTCTGACCGATGC
CGCGCGGCGACGGCCTATCAACCTGCTCGCCCGCGTCGAGCAGGGCCTATGCGA
CGTTCTCGGCGGCGATGCATCCTACGGGCACCGGATCACAAAGAACCCGCTCAG
CACCGCCCATGCGACCCTCTGGGGCCCCGCAGACGCGCTCTACGAGCTGCGCGC

CCTCGCACACACCCTCGACGAGATCCACGCACTGCCGGAGGCAGGGAACCCGCG
TCGCAACGTCACCCGATCAACGGTCGGCCGCAACGTCACCCTGTTCGACACCACC
CGCATGTGGGCATACCGGGCCGTCCGGCACTCCTGGGGCGGCCCGGTCGCCGA
ATGGGAGCACACCGTATTCGAGCACATCCACCTACTGAACGAGACGATCATCGCC
GACGAATTCGCCACAGGCCCCCTCGGCTTGAACGAACTTAAGCACTTATCTCGATC
CATTTCCCGATGGGTCTGGCGCAACTTCACCCCCGAAACCTTCCGCGCACGCCAG
AAAGCGATCAGCCTCCGTGGAGCATCCAAAGGCGGCAAAGAAGGCGGCCACAAA
GGCGGCATTGCCAGTGGCGCATCACGGCGCGCCCATACCCGTCAACAGTTCTTG
GAGGGTCTCTCATGACCACACGTGAACGTCTCCCCGCAACGGCTACAGCATCGC
CGCTGCTGCGAAAAGCTCGGTGTCTCCGAGTCCACCGTCAAGCGGTGGACTTCC
GAGCCACGCGAGGAGTTCGTGGCCCGCGTTGCCGCACGCCACGCGCGGATTCGT
GAGCTgCGCTCGGAGGGTCAGAGCATGCGTGCGATTGCTGCCGAGGTCGGGGTT
TCCGTGGGCACCGTGCACTACGCGCTGAACAAGAATCGAACTGACGCATGACCGT
AACGCCGCACGATGAGCATTTTCTTGATCGTGCACCGCTTGGCACTACGTTCGCGT
GCGGTTGCACAGTGCGCGCCACGTTCTTATCCTGCGGCCATTGTGGCTACAGCCA
ATGGGGGGCATCAGCAACGGACGTTGAACCCGGTGGGCAAGTGTTACTCAGGGG
GACATGCCCAGTCTGCGGCGCTCGGATTGACGGTATGGCAGTCGTGCATGCGGC
CCCACCGTCAAACTCATTCAGGTATCAGTGAGAACCCTCATGGCACCCCTCGTG
ACACGTTCTCGTTGCGATCAGCTGCGGCCAAGCTTGATCTTCGGGCAGCGTTGGG
TCCTGGCCACGGGTGCGCATGATCGTGCTCCTGTCGTTGAGGACCCGGCTAGGCT
GGCGGGGTTGCCTTACTGGTTAGCAGAATGAATCACCGATACGCGAGCGAACGTG
AAGCGACTGCTGCTGCAAAACGTCTGCGACCTGAGCAACAACATGAATGGTCTTC
GGTTTCCGTGTTTCGTAAAGTCTGGAAACGCGGAAGTCAGCGCCCTGCACCATTAT
GTTCCGGATCTGCATCGCAGGATGCTGCTGGCTACCCTGTGGAACACCTACATCT
GTATTAACGAAGCGCTGGCATTGACCCTGAGTGATTTTTCTCTGGTCCCGCCGCAT
CCATACCGCCAGTTGTTTACCCTCACAACGTTCCAGTAACCGGGCATGTTCATCAT
CAGTAACCCGTATCGTGAGCATCCTCTCTCGTTTCATCGGTATCATTACCCCCATG
AACAGAAATCCCCCTTACACGGAGGCATCAGTGACCAAACAGGAAAAAACCGCCC
TTAACATGGCCCGCTTTATCAGAAGCCAGACATTAACGCTTCTGGAGAAACTCAAC
GAGCTGGACGCGGATGAACAGGCAGACATCTGTGAATCGCTTCACGACCACGCTG
ATGAGCTTTACCGCAGCTGCCTCGCGCGTTTCGGTGATGACGGTGAAAACCTCTG

ACACATGCAGCTCCCGGAGACGGTCACAGCTTGTCTGTAAGCGGATGCCGGGAG

CAGACAAGCCCGTCAGGGCGCGTCAGCGGGTGTTGGCGGGTGTCGGGGCGCAG

CCATGACCCAGTCACGTAGCGATAGCGGAGTGTATACTGGCTTAACTATGCGGCA

TCAGAGCAGATTGTACTGAGAGTGCACCATATGCGGTGTGAAATACCGCACAGAT

GCGTAAGGAGAAAATACCGCATCAGGCGCTCTTCCGCTTCCTCGCTCACTGACTC

GCTGCGCTCGGTCGTTCGGCTGCGGCGAGCGGTATCAGCTCACTCAAAGGCGGT

AATACGGTTATCCACAGAATCAGGGGATAACGCAGGAAAGAACATGTGAGCAAAA

GGCCAGCAAAAGGCCAGGAACCGTAAAAAGGCCGCGTTGCTGGCGTTTTTCCATA

GGCTCCGCCCCCCTGACGAGCATCACAAAAATCGACGCTCAAGTCAGAGGTGGC

GAAACCCGACAGGACTATAAAGATACCAGGCGTTTCCCCCTGGAAGCTCCCTCGT

GCGCTCTCCTGTTCCGACCCTGCCGCTTACCGGATACCTGTCCGCCTTTCTCCCTT

CGGGAAGCGTGGCGCTTTCTCATAGCTCACGCTGTAGGTATCTCAGTTCGGTGTA

GGTCGTTCGCTCCAAGCTGGGCTGTGTGCACGAACCCCCCGTTCAGCCCGACCG

CTGCGCCTTATCCGGTAACTATCGTCTTGAGTCCAACCCGGTAAGACACGACTTAT

CGCCACTGGCAGCAGCCACTGGTAACAGGATTAGCAGAGCGAGGTATGTAGGCG

GTGCTACAGAGTTCTTGAAGTGGTGGCCTAACTACGGCTACACTAGAAGGACAGT

ATTTGGTATCTGCGCTCTGCTGAAGCCAGTTACCTTCGGAAAAAGAGTTGGTAGCT

CTTGATCCGGCAAACAAACCACCGCTGGTAGCGGTGGTTTTTTTGTTTGCAAGCAG

CAGATTACGCGCAGAAAAAAGGATCTCAAGAAGATCCTTTGATCTTTTCTACGGG

GTCTGACGCTCAGTGGAACGAAAACTCACGTTAAGGGATTTTGGTCATGAGATTAT

CAAAAAGGATCTTCACCTAGATCCTTTTAAATTAAAAATGAAGTTTTAAATCAATCTA

AAGTATATATGAGTAAACTTGGTCTGACAGTTACCAATGCTTAATCAGTGAGGCAC

CTATCTCAGCGATCTGTCTATTTCGTTCATCCATAGTTGCCTGACTCCCCGTCGTGT

AGATAACTACGATACGGGAGGGCTTACCATCTGGCCCCAGTGCTGCAATGATACC

GCGAGACCCACGCTCACCGGCTCCAGATTTATCAGCAATAAACCAGCCAGCCGGA

AGGGCCGAGCGCAGAAGTGGTCCTGCAACTTTATCCGCCTCCATCCAGTCTATTA

ATTGTTGCCGGGAAGCTAGAGTAAGTAGTTCGCCAGTTAATAGTTTGCGCAACGTT

GTTGCCATTGCTGCAGGCATCGTGGTGTCACGCTCGTCGTTTGGTATGGCTTCATT

CAGCTCCGGTTCCCAACGATCAAGGCGAGTTACATGATCCCCCATGTTGTGCAAAA

AAGCGGTTAGCTCCTTCGGTCCTCCGATCGTTGTCAGAAGTAAGTTGGCCGCAGT

GTTATCACTCATGGTTATGGCAGCACTGCATAATTCTCTTACTGTCATGCCATCCGT

AAGATGCTTTTCTGTGACTGGTGAGTACTCAACCAAGTCATTCTGAGAATAGTGTAT
GCGGCGACCGAGTTGCTCTTGCCCGGCGTCAACACGGGATAATACCGCGCCACA
TAGCAGAACTTTAAAAGTGCTCATCATTGGAAAACGTTCTTCGGGGCGAAAACTCT
CAAGGATCTTACCGCTGTTGAGATCCAGTTCGATGTAACCCACTCGTGCACCCAAC
TGATCTTCAGCATCTTTTACTTTCACCAGCGTTTCTGGGTGAGCAAAAACAGGAAG
GCAAAATGCCGCAAAAAAGGGAATAAGGGCGACACGGAAATGTTGAATACTCATA
CTCTTCCTTTTTCAATATTATTGAAGCATTTATCAGGGTTATTGTCTCATGAGCGGAT
ACATATTTGAATGTATTTAGAAAAATAAACAAATAGGGGTTCCGCGCACATTTCCCC
GAAAAGTGCCACCTGACGTCTAAGAAACCATTATTATCATGACATTAACCTATAAAA
ATAGGCGTATCACGAGGCCCTTTCGTCTTCAAGAATTCAAGCTTGTCGACGTTAAC
CTGCAGGCATGCGGATCCGGTACCAGCTGTCTCTTATACACATCTCTGCAGTTAAC
GCTGACCCGGACCACCACGACGACCCGGACGACCACCGGTACGACCACCACCAG
ACGGACCGCGGCGACCACCCTGGTCACCCTGTTCACGTTCACCGGTACGACCGTC
GTCACGGTTACGACCAGAAGAACGACGGTCACCGTGACCAGACGCTTGCCGGTCT
CTGCGATCTTGGCCCCCGGTACGTCTGTCACCACGGTCCTGACCACCGGTACGAC
GGTCGCCCCTATCCTGTCCACCCGTCCTACGACCACGCTGCGGCTGACCACCACG
CGGTTCGTTTTTCTGGTCCAGACGTTCGAACAGACGCAGCCACTGTTCCGGACGA
ACGTAAGCAACAACAGCACCACCACCAACACCAGCGTGGTCCAGAGCAGCTTCAA
CACGACGACGCGGGTATTTACGCAGCAGAGAAGCCTGGATGTTACCACCAACACC
GGTGAAGCACAGTTCAACCATAGATTCGTAACGTTCCAGAGCAGCACCTTCCAGCA
GCGGTTCAGCACGACGACGCAGACGCATGATAGCAGAGTCAACTTTCGGAACCGG
TTTGAACAGACGACGATCGACTTTTTCAACGAATTCCCATTCGAACAGCGGCCAGG
TCATAACGGTCAGACGAGACCAACGACCGTAGTCACCGGTACGTTTACGAGCGAA
TTCCAGCTGGGTAACCATGGTAGCGGTTTCGATGGTCGGAGCTTCCAGGCACCAG
TCAACGATAGCAGAGGTGATACCGTACGGGATAGCACCAACGAAAGCGAACGGTT
CCGGCGGCGGTTCAGCGGTCAGGAAGTCAGCGTTAACAACTTCGATGTTCGGGTG
ACCAGACAGTTTTTCACGCAGAGATTTAGCCAGACGCGGGTCGATTTCGTAAGAG
GTAACCTGACGAGCACGGTCAGCCAGTTCACGGGTCAGCAGACCTTCACCCGGAC
CAGCTTCCAGAACCGGCAGGTCCGGACGCAGTTCAGCGGTTTCAGCGATACGAG
CGATGGTTTTACGGTCACGCAGGAAGTTCTGGCCGAATTGGCGGCGGTTACGGTC
ACGCTCGGTACGGCCGAGGACGGGGCGATTCTGGTTGGGGTGCTGGCGGTCTTG

GTTGCGGCGGCGGGGGCGGGGCTGTTCATCGGAGGAGGACATGACGATCTCCC
GATGTGTCGTACGTATGGGAATTGACGAAAATCATATAGATTTTCAGTTGAAGTGTA
GCACTGCATAGTGTCATCGAAGTGTCGGCGGCAACGGTATGCCCGGTAGGGGGT
GGGTCTGCCTTTCGGTTTCGTGAACCTGATGCGGCGACGCGCATGTAGCCGATAA
CCCCTAGACTCGCCCGTGCAGATCGTCGGTGTGGATCCAGATGTGTATAAGAGAC
AGCTGGTACCGGCCGCTGCT

pEND-274 of SEQ ID NO 14:

GTGCGTGCGGGCGTGAGCGTTTCTACGCTGCGGCGCAGGAAATCAGAGCTTGAG
GCTGCCGGAGCGACGGTAGACCCGTCCGGTTGGGTGGTGCCACTGCGTGCACTC
AAGGTCGTTTTTGGGGTGTCAGATGAGACCTCGAATGCGAATACCAGTAAATCCTG
ACCTGTGAGTTGTGAATTCATAGGATAGATTCTGGAAACTTTACCGTCCGAGCTCC
AGCCTGCGGTCCGGCCTGTCCTGATCATGATCCGTCCGGGGGCCGCTAGTTGGG
CGGTCCCCGGACGATTTTGTGTACCCGCAGATGCGTGCCAGACAAGCGCCTCGG
CTGTTTTGTGGGTTTGTGTTGGTGGTGGGGGTGTGTGTAGTGTGTGTTGGGCCGT
TGGGGGCTGCGTGTGTGGTTCTTGGTGGTGGCTGTGTTTGGGATGTCGCTGGGAC
GCCCGTAGAACTCTGTGAGGATAAAGTCTCCCTTACAGCCTGCGGTCCGGTAATTT
TGTTTAACTTTAAGAAGGAGATATACATatgttcgtccaaatcgctgccagcctggcagccgcatcgtcc
attgcactcggcataccaggagctgccGAGCAGGAAGTGCCGATGAACCTGGTCTCGGCTGAT
GGCAAGGAGGTTTCCATCGGGAAGATCACCATCCAGGAGACCCCATACGGTCTGT
TGTTCACCCCCGCCCTGCACTCGCTGAGCGAGGGTATTCACGGTTTCCACGTCCA
CGAGAAGGGCAACTGCGCCCCGGCGCTTAAAGACGGCAAGCCGGTCGCCGCGCT
GTCCGCCGGTGGTCACTTTGACCCGAAGAATACTGGCAAGCACCTGGGCCCGTG
GTCCCCTGACGGCCACcTcGGtGACCTGCCGGCCCTGTTCGTCACCCATGACGGCA
AAGCCAACTACCCGGTCCTGGCCCCGCGCCTCAACTCCCTGAAGGAGATCAAGG
GCCGCTCcCTcATGCTcCACGCtGGtGGCGACAATCACCACGACCACCCAGAGCCC
CTGGGCGGTGGCGGtGCCCGCATGGCCTGCGGCATTATCCAGCATCATCACCATC
ACCACTGAtaaTCGCTGGGACGCCCGCTCGAGCAATAAACAGTTGATAGGGCTTCT
CCGTTACCATGGTTCAGCCAAAAAACTTAAGACCGCCGGTCTTGTCCACTACCTTG
CAGTAATGCGGTGGACAGGATCGGCGGTTTTCTTTTCTCTTCTCAAGCTAGCGAAA

TCCCTGAAACTGAGACTGTAGAAAATAAGCTTCCGAAACTGAACCTGTTATTTTACG

CTACGGAcgCCCGGTCATGACGCTGAGTTAGTGGCGCAGCTGCGCTCTGAGAACG

AGTTTTTACGGCGTCAGGTCGAGCAGCAGGCGCGCACGATCGAACGGCAGGCTG

AGGCACACGCGGTGGTCTCAGCGCAGCTCACACGGGTTGGCCAGCTTGAGGCCG

GCGACGCAGCAGCACCGACACTGGCACCCGTTGAAAGGCCGGCTCCGCGACGGC

GGTGGTGGCAGCGTCGGTAGCGGTCAGGATCGCTCTGGCGTGACGAGTGTGTCT

GGCAGTGCGAACAGTTGCTCGACCAGTGGCAGCAGAAGCGAGATCGCTGCGTGG

TGCTGTTCCTCGGTCAGTTCGTCGAGGACTGGCGGGTCTTGCTGCGTCCAGCCGA

TCGCCTCGGCGGCCAAGGTCAGTTCCAAGCTGTGCCAACGCACACGCCCCTCGG

CTGACAGCTGAGTCTCGAACTGTGCAACTGGACCGGCCGGAAGATGCACGTTGCC

GAGGTCGTGAGTGGCCAAGCGCACGTCAAAGAGTGCTGCTTCGTAGCCGCGCAG

AAATGGCAGTGCTCGGTCGATTCGGATCGGCCTGCCCAGGTACATTCCGGGCCGC

TTGATGAACGCCTCCGCGTAGAAGCGCACCGTTCTCGGCCCGGCCTCGTGATCTG

TCACTGTGCACGCTCCTCTCGATGGTTCTCGACGCTACCGGAGACCACCGACGTT

CATGCCCAGCGCAGCGACCTGAAAGGACCAAGCCGAGTTAGCCGTGCTAACCGTA

TAGCTTGCTCCGTCGCCTCTGAGGGCAACCACCTGCGCAGCAGGTGGGCGGCAG

CCCGCGCGCAAGCGCCTACCGGGTTTGGGCACAGCCCATAAATCAACGCCTCCG

GTGTTGAAGCGATCGTGTGTCACGATTGCTATGCTTGCTACCCCTTCAGGGTTTTC

GTATACACAAATCAAGTTTTTTCGTATACGCTAATGCCATGAGTGAGCATCTACTGC

ACGGCAAGCCCGTCACCAACGAGCAGATTCAGGCATGGGCAGACGAGGCCGAGG

CCGGATACGACCTGCCCAAACTCCCCAAGCCACGGCGCGGACGCCCGCCCGTAG

GAGACGGTCCGGGCACCGTCGTACCCGTGCGTCTCGACGCGGCCACCGTTGCCG

CTCTCACAGAACGAGCAACAGCCGAGGGCATCACGAACCGTTCAGACGCGATCCG

AGCCGCAGTCCACGAGTGGACACGGGTTGCCTGACCTCCACGACTCAGCACGCA

AGCACTACCAACGAGACCGGCTCGACGACACGGCCGTGCTCTACGCGGCCACCC

ACGTTCTCAACTCCCGGCCACTCGACGACGAAGACGACCCGCGCCGCTGGCTCAT

GATCGGAACCGACCCAGCAGGCCGCCTACTCGAACTCGTCGCACTGATCTACGAC

GACGGCTACGAACTGATCATCCACGCAATGAAAGCCCGCACCCAATACCTCGACC

AGCTCTAACCAAGAAAGGAACCTGATGAGCGACCAGCTAGACAGCGACCGCAACT

ACGACCCGATGATCTTCGACGTGATGCGCGAGACCGCGAACCGCGTCGTCGCCA

CGTACGTTGCATGGGAAGATGAAGCCGCTGATCCCCGCGAGGCTGCGCACTGGC

AGGCCGAGCGATTCCGCACCCGGCACGAGGTGCGCGCCGTCGACCCTGACAGCC
GGCGAGCAGTTCAGGCGAAGATCGCACAGCTGCGCGAGGAACTAGCCGCAATGC
CCGAACACGCCCCAGCCATCCCTTGGAGCAGGTGGCAGCGTCAGGGGAGTCGGG
GGATGTTTGGCAGGGGATGTGGAAAGAGAGTTCGCTTTGCTCACATGGCTCAACC
GGGTAACTAACTGATATGGGGTCTTCGTCGCCCACTTTGAACACGCCGAGGAATG
GACCACGCTGAACGTGACTCGCATGCTTCACTGCATGTATGGATTCGTTCGAGAC
GTTGTTCCCTGAGAGCTGGCTGCCACGCAAGCCGCTGGCGTCAGCCGAGAAGTCT
GGGGCGTACCGGCACGTGACTCGGCAGAGGGCGCTGGAGCTGCCTTACATCGAA
GCGAACCCGTTGGTCATGCAGTCCTTGGTCATCACCGATCGAGATGCTTCGGATG
CTGACTGGGCCGCAGACCTCGCTGGGCTGCCTTCACCGTCCTACGTGTCCATGAA
CCGTGTCACGACCACCGGACACATCGTCTATGCCTTGAAGAACCCTGTGTGTCTG
ACCGATGCCGCGCGGCGACGGCCTATCAACCTGCTCGCCCGCGTCGAGCAGGGC
CTATGCGACGTTCTCGGCGGCGATGCATCCTACGGGCACCGGATCACAAAGAACC
CGCTCAGCACCGCCCATGCGACCCTCTGGGGCCCCGCAGACGCGCTCTACGAGC
TGCGCGCCCTCGCACACACCCTCGACGAGATCCACGCACTGCCGGAGGCAGGGA
ACCCGCGTCGCAACGTCACCCGATCAACGGTCGGCCGCAACGTCACCCTGTTCGA
CACCACCCGCATGTGGGCATACCGGGCCGTCCGGCACTCCTGGGGCGGCCCGGT
CGCCGAATGGGAGCACACCGTATTCGAGCACATCCACCTACTGAACGAGACGATC
ATCGCCGACGAATTCGCCACAGGCCCCCTCGGCTTGAACGAACTTAAGCACTTAT
CTCGATCCATTTCCCGATGGGTCTGGCGCAACTTCACCCCCGAAACCTTCCGCGC
ACGCCAGAAAGCGATCAGCCTCCGTGGAGCATCCAAAGGCGGCAAAGAAGGCGG
CCACAAAGGCGGCATTGCCAGTGGCGCATCACGGCGCGCCCATACCCGTCAACA
GTTCTTGGAGGGTCTCTCATGACCACACGTGAACGTCTCCCCCGCAACGGCTACA
GCATCGCCGCTGCTGCGAAAAGCTCGGTGTCTCCGAGTCCACCGTCAAGCGGT
GGACTTCCGAGCCACGCGAGGAGTTCGTGGCCCGCGTTGCCGCACGCCACGCGC
GGATTCGTGAGCTgCGCTCGGAGGGTCAGAGCATGCGTGCGATTGCTGCCGAGG
TCGGGGTTTCCGTGGGCACCGTGCACTACGCGCTGAACAAGAATCGAACTGACGC
ATGACCGTAACGCCGCACGATGAGCATTTTCTTGATCGTGCACCGCTTGGCACTAC
GTTCGCGTGCGGTTGCACAGTGCGCGCCACGTTCTTATCCTGCGGCCATTGTGGC
TACAGCCAATGGGGGGCATCAGCAACGGACGTTGAACCCGGTGGGCAAGTGTTA
CTCAGGGGGACATGCCCAGTCTGCGGCGCTCGGATTGACGGTATGGCAGTCGTG

CATGCGGCCCCACCGTCAAACTCATTCAGGTATCAGTGAGAACCCTCATGGCACC

CCCTCGTGACACGTTCTCGTTGCGATCAGCTGCGGCCAAGCTTGATCTTCGGGCA

GCGTTGGGTCCTGGCCACGGGTGCGCATGATCGTGCTCCTGTCGTTGAGGACCC

GGCTAGGCTGGCGGGGTTGCCTTACTGGTTAGCAGAATGAATCACCGATACGCGA

GCGAACGTGAAGCGACTGCTGCTGCAAACGTCTGCGACCTGAGCAACAACATGA

ATGGTCTTCGGTTTCCGTGTTTCGTAAAGTCTGGAAACGCGGAAGTCAGCGCCCT

GCACCATTATGTTCCGGATCTGCATCGCAGGATGCTGCTGGCTACCCTGTGGAAC

ACCTACATCTGTATTAACGAAGCGCTGGCATTGACCCTGAGTGATTTTTCTCTGGT

CCCGCCGCATCCATACCGCCAGTTGTTTACCCTCACAACGTTCCAGTAACCGGGC

ATGTTCATCATCAGTAACCCGTATCGTGAGCATCCTCTCTCGTTTCATCGGTATCAT

TACCCCCATGAACAGAAATCCCCCTTACACGGAGGCATCAGTGACCAAACAGGAA

AAAACCGCCCTTAACATGGCCCGCTTTATCAGAAGCCAGACATTAACGCTTCTGGA

GAAACTCAACGAGCTGGACGCGGATGAACAGGCAGACATCTGTGAATCGCTTCAC

GACCACGCTGATGAGCTTTACCGCAGCTGCCTCGCGCGTTTCGGTGATGACGGTG

AAAACCTCTGACACATGCAGCTCCCGGAGACGGTCACAGCTTGTCTGTAAGCGGA

TGCCGGGAGCAGACAAGCCCGTCAGGGCGCGTCAGCGGGTGTTGGCGGGTGTC

GGGGCGCAGCCATGACCCAGTCACGTAGCGATAGCGGAGTGTATACTGGCTTAAC

TATGCGGCATCAGAGCAGATTGTACTGAGAGTGCACCATATGCGGTGTGAAATAC

CGCACAGATGCGTAAGGAGAAAATACCGCATCAGGCGCTCTTCCGCTTCCTCGCT

CACTGACTCGCTGCGCTCGGTCGTTCGGCTGCGGCGAGCGGTATCAGCTCACTCA

AAGGCGGTAATACGGTTATCCACAGAATCAGGGGATAACGCAGGAAAGAACATGT

GAGCAAAAGGCCAGCAAAAGGCCAGGAACCGTAAAAAGGCCGCGTTGCTGGCGT

TTTTCCATAGGCTCCGCCCCCCTGACGAGCATCACAAAAATCGACGCTCAAGTCAG

AGGTGGCGAAACCCGACAGGACTATAAAGATACCAGGCGTTTCCCCCTGGAAGCT

CCCTCGTGCGCTCTCCTGTTCCGACCCTGCCGCTTACCGGATACCTGTCCGCCTT

TCTCCCTTCGGGAAGCGTGGCGCTTTCTCATAGCTCACGCTGTAGGTATCTCAGTT

CGGTGTAGGTCGTTCGCTCCAAGCTGGGCTGTGTGCACGAACCCCCGTTCAGCC

CGACCGCTGCGCCTTATCCGGTAACTATCGTCTTGAGTCCAACCCGGTAAGACAC

GACTTATCGCCACTGGCAGCAGCCACTGGTAACAGGATTAGCAGAGCGAGGTATG

TAGGCGGTGCTACAGAGTTCTTGAAGTGGTGGCCTAACTACGGCTACACTAGAAG

GACAGTATTTGGTATCTGCGCTCTGCTGAAGCCAGTTACCTTCGGAAAAAGAGTTG

GTAGCTCTTGATCCGGCAAACAAACCACCGCTGGTAGCGGTGGTTTTTTTGTTTGC
AAGCAGCAGATTACGCGCAGAAAAAAGGATCTCAAGAAGATCCTTTGATCTTTTC
TACGGGGTCTGACGCTCAGTGGAACGAAAACTCACGTTAAGGGATTTTGGTCATG
AGATTATCAAAAAGGATCTTCACCTAGATCCTTTTAAATTAAAAATGAAGTTTTAAAT
CAATCTAAAGTATATATGAGTAAACTTGGTCTGACAGTTACCAATGCTTAATCAGTG
AGGCACCTATCTCAGCGATCTGTCTATTTCGTTCATCCATAGTTGCCTGACTCCCC
GTCGTGTAGATAACTACGATACGGGAGGGCTTACCATCTGGCCCCAGTGCTGCAA
TGATACCGCGAGACCCACGCTCACCGGCTCCAGATTTATCAGCAATAAACCAGCC
AGCCGGAAGGGCCGAGCGCAGAAGTGGTCCTGCAACTTTATCCGCCTCCATCCAG
TCTATTAATTGTTGCCGGGAAGCTAGAGTAAGTAGTTCGCCAGTTAATAGTTTGCG
CAACGTTGTTGCCATTGCTGCAGGCATCGTGGTGTCACGCTCGTCGTTTGGTATG
GCTTCATTCAGCTCCGGTTCCCAACGATCAAGGCGAGTTACATGATCCCCCATGTT
GTGCAAAAAAGCGGTTAGCTCCTTCGGTCCTCCGATCGTTGTCAGAAGTAAGTTGG
CCGCAGTGTTATCACTCATGGTTATGGCAGCACTGCATAATTCTCTTACTGTCATGC
CATCCGTAAGATGCTTTTCTGTGACTGGTGAGTACTCAACCAAGTCATTCTGAGAAT
AGTGTATGCGGCGACCGAGTTGCTCTTGCCCGGCGTCAACACGGGATAATACCGC
GCCACATAGCAGAACTTTAAAAGTGCTCATCATTGGAAAACGTTCTTCGGGGCGAA
AACTCTCAAGGATCTTACCGCTGTTGAGATCCAGTTCGATGTAACCCACTCGTGCA
CCCAACTGATCTTCAGCATCTTTTACTTTCACCAGCGTTTCTGGGTGAGCAAAAACA
GGAAGGCAAAATGCCGCAAAAAGGGAATAAGGGCGACACGGAAATGTTGAATAC
TCATACTCTTCCTTTTTCAATATTATTGAAGCATTTATCAGGGTTATTGTCTCATGAG
CGGATACATATTTGAATGTATTTAGAAAAATAAACAAATAGGGGTTCCGCGCACATT
TCCCCGAAAAGTGCCACCTGACGTCTAAGAAACCATTATTATCATGACATTAACCTA
TAAAAATAGGCGTATCACGAGGCCCTTTCGTCTTCAAGAATTCAAGCTTGTCGACG
TTAACCTGCAGGCATGCGGATCCGGTACCAGCTGTCTCTTATACACATCTCTGCAG
TTAACGCTGACCCGGACCACCACGACGACCCGGACGACCACCGGTACGACCACC
ACCAGACGGACCGCGGCGACCACCCTGGTCACCCTGTTCACGTTCACCGGTACG
ACCGTCGTCACGGTTACGACCAGAAGAACGACGGTCACCGTGACCAGACGCTTGC
CGGTCTCTGCGATCTTGGCCCCCGGTACGTCTGTCACCACGGTCCTGACCACCGG
TACGACGGTCGCCCCTATCCTGTCCACCCGTCCTACGACCACGCTGCGGCTGACC
ACCACGCGGTTCGTTTTTCTGGTCCAGACGTTCGAACAGACGCAGCCACTGTTCC

GGACGAACGTAAGCAACAACAGCACCACCACCAACACCAGCGTGGTCCAGAGCA
GCTTCAACACGACGACGCGGGTATTTACGCAGCAGAGAAGCCTGGATGTTACCAC
CAACACCGGTGAAGCACAGTTCAACCATAGATTCGTAACGTTCCAGAGCAGCACCT
TCCAGCAGCGGTTCAGCACGACGACGCAGACGCATGATAGCAGAGTCAACTTTCG
GAACCGGTTTGAACAGACGACGATCGACTTTTTCAACGAATTCCCATTCGAACAGC
GGCCAGGTCATAACGGTCAGACGAGACCAACGACCGTAGTCACCGGTACGTTTAC
GAGCGAATTCCAGCTGGGTAACCATGGTAGCGGTTTCGATGGTCGGAGCTTCCAG
GCACCAGTCAACGATAGCAGAGGTGATACCGTACGGGATAGCACCAACGAAAGCG
AACGGTTCCGGCGGCGGTTCAGCGGTCAGGAAGTCAGCGTTAACAACTTCGATGT
TCGGGTGACCAGACAGTTTTTCACGCAGAGATTTAGCCAGACGCGGGTCGATTTC
GTAAGAGGTAACCTGACGAGCACGGTCAGCCAGTTCACGGGTCAGCAGACCTTCA
CCCGGACCAGCTTCCAGAACCGGCAGGTCCGGACGCAGTTCAGCGGTTTCAGCG
ATACGAGCGATGGTTTTACGGTCACGCAGGAAGTTCTGGCCGAATTGGCGGCGGT
TACGGTCACGCTCGGTACGGCCGAGGACGGGGCGATTCTGGTTGGGGTGCTGGC
GGTCTTGGTTGCGGCGGCGGGGGCGGGGCTGTTCATCGGAGGAGGACATGACG
ATCTCCCGATGTGTCGTACGTATGGGAATTGACGAAAATCATATAGATTTTCAGTTG
AAGTGTAGCACTGCATAGTGTCATCGAAGTGTCGGCGGCAACGGTATGCCCGGTA
GGGGGTGGGTCTGCCTTTCGGTTTCGTGAACCTGATGCGGCGACGCGCATGTAG
CCGATAACCCCTAGACTCGCCCGTGCAGATCGTCGGTGTGGATCCAGATGTGTAT
AAGAGACAGCTGGTACCGGCCGCTGCT

pEND-387 of SEQ ID NO 17:

tctagaGGAAATCAGAGCTTGAGGCTGCCGGAGCGACGGTAGACCCGTCCGGTTGG
GTGGTGCCACTGCGTGCACTCAAGGTCGTTTTTGGGGTGTCAGATGtGACCTCGAA
TGCGGAATTCATAGGATAGATTCTGGAAACTTTACCGTCCGAGCTCCAGCCTGCG
GTCCGGTTGACACCCTATCAGTGATAGAGTATAATGCTAGCTCGCTGGGACGCCC
GTAGAACTCTGTGAGGATAAAGTCTCCCTTACAGCCTGCGGTCCGGTAATTTTGTT
TAACTTTAAGAAGGAGATATACATatgttcgtccaaatcgctgccagcctggcagccgcatcgtccattgca
ctcggcataccaggagctgccGAGCAGGAAGTGCCGATGAACCTGGTCTCGGCTGATGGCA
AGGAGGTTTCCATCGGGAAGATCACCATCCAGGAGACCCCATACGGTCTGTTGTT

CACCCCCGCCCTGCACTCGCTGAGCGAGGGTATTCACGGTTTCCACGTCCACGAG

AAGGGCAACTGCGCCCCGGCGCTTAAAGACGGCAAGCCGGTCGCCGCGCTGTCC

GCCGGTGGTCACTTTGACCCGAAGAATACTGGCAAGCACCTGGGCCCGTGGTCC

CCTGACGGCCACcTcGGtGACCTGCCGGCCCTGTTCGTCACCCATGACGGCAAAG

CCAACTACCCGGTCCTGGCCCCGCGCCTCAACTCCCTGAAGGAGATCAAGGGCC

GCTCcCTcATGCTcCACGCtGGtGGCGACAATCACCACGACCACCCAGAGCCCCTG

GGCGGTGGCGGtGCCCGCATGGCCTGCGGCATTATCCAGCATCATCACCATCACC

ACTGAtaaTCGCTGGGACGCCCGCTCGAGCAATAAACAGTTGATAGGGCTTCTCCG

TTACCATGGTTCAGCCAAAAAACTTAAGACCGCCGGTCTTGTCCACTACCTTGCAG

TAATGCGGTGGACAGGATCGGCGGTTTTCTTTTCTCTTCTCAATTCTTCTGACCTGT

AACGAATAATAGATAGTAAAGTAGTCTCCGATTGAGTTTTCTCTGCCGAGTCCCAC

CCAGTTCTGTGATTTCAGTAAGTTGGTAATTGATACACTGTTGCGAGAACTGgTGCC

TGGTAGTAGATAGGTTGTTATTGAGTAAGAAGGTAAAGTGAACGAAATCCCTGAAA

CTGAGACTGTAGAAAATAAGCTTGTCCAGACTATTGGATCCAAGAGATTTCTACAC

GATTGAGCACTGTCTCCTGCAGGCTCGGTACCAAATTCCAGAAAAGAGGCCTCCC

GAAAGGGGGGCCTTTTTTCGTTTTGGTCCTAATAGATAAAGGATAGGTCTGGTAGT

GTTGTTCGTTCTCGCAGGTAAATCAATAATACTCtGCAGTTCCGTAGACTTTTCAGT

GGGACAGGGTAGCGATAACAGATAGATTGTAATAAGACACAGTAGGTGCTCGTAG

TTGCGTGAAGAGAACCGCTCAGGAAATCCAGTCAGAAGTATTGGTAATCGTTGAAA

ACTCAGTCGACCAGCCTGCGGTCCGGacaccgacgatctgcacgggcgagtctaggggttatcggct

acatgcgcgtcgccgcatcaggttcacgaaaccgaaaggcagacccaccccctaccgggcataccgttgccgccgac

acttcgatgacactatgcagtgctacacttcaactgaaaatctatatgattttcgtcaattcTCGCTGGGACGCCCG

TAGAACTCTGTGAGGATAAAGTCTCCCTTACAGCCTGCGGTCCGGTAATTTTGTTT

AACTTTAAGAAGGAGATATACATATGTCAAGACTAGATAAGAGCAAGGTCATTAACA

GCGCACTGGAGTTACTAAACGAAGTTGGAATAGAGGGATTGACTACACGAAAATTA

GCACAGAAATTAGGAGTAGAACAACCGACACTTTATTGGCACGTTAAAAATAAAAG

AGCTTTGCTGGATGCTTTAGCTATAGAAATGCTAGACCGACACCACACATTTTT

GCCCCTTAGAAGGAGAAAGTTGGCAAGATTTCCTACGTAACAACGCCAAATCATTT

AGATGCGCGCTATTATCACACCGCGACGGCGCCAAAGTTCACCTCGGGACAAGAC

CTACGGAGAAGCAATACGAAACCCTAGAGAATCAATTAGCCTTTCTATGTCAACAA

GGATTCAGTTTGGAAAACGCCCTCTACGCCCTATCAGCCGTTGGTCATTTTACATT

GGGATGCGTTTTAGAGGATCAGGAACATCAAGTAGCAAAAGAAGAAAGAGAGACA

CCCACCACCGACAGCATGCCACCATTATTACGTCAAGCTATTGAACTATTCGATCA

TCAGGGAGCGGAACCGGCATTTTTATTTGGCCTCGAATTAATCATATGTGGTTTAG

AAAAACAATTAAAGTGTGAATCTGGATCCCATCATCACCATCACCACTGAtaaTCGCT

GGGACGCCCGGACGTCCTATTACACTCGTCGTTGGAAACTGAAGATGCGGCCGCg

gaaacacagAAAAAAGCCCGCACCTGACAGTGCGGGCTTTTTTTTTcgaccaaaggTAGC

GAACGACGAGTCACTGTTGAGGATAAATACTTTCTCTACTAGCTAGCTGTTACACA

GGTCCTCAGCGGCGCGCCcgCCCGGTCATGACGCTGAGTTAGTGGCGCAGCTGC

GCTCTGAGAACGAGTTTCGACCCTGACAGCCGGCGAGCtGTTCAGGCGAAGATCG

CACAGCTGCGCGAGGAACTAGCCGCAATGCCCGAACACGCCCCAGCCATCCCTT

GGAGCAGGTGGCAGCGTCAGGGGAGTCGGGGGATGTTTGGCAGGGGATGTGGA

AAGAGAGTTCGCTTTGCTCACATGGCTCAACCGGGTAACTAACTGATATGGGcTCT

TCGTCGCCCACTTTGAACACGCCGAGGAATGGACCACGCTGAACGTGACTCGCAT

GCTTCACTGCATGTATGGATTCGTTCGAGACtTTGTTCCCTGAGAGCTGGCTGCCA

CGCAAGCCGCTGGCGTCAGCCGAGAAGTCTGGGGCGTACCGGCACGTGACTCGG

CAGAGGGCGCTGGAGCTGCCTTACATCGAAGCGAACCCGTTGGTCATGCAGTCCT

TGGTCATCACCGATCGAGATGCTTCGGATGCTGACTGGGCCGCAGACCTCGCTGG

GCTGCCTTCACCGTCCTACGTGTCCATGAACCGTGTCACGACCACCGGACACATC

GTCTATGCCTTGAAGAACCCTGTGTGTCTGACCGATGCCGCGCGGCGACGGCCTA

TCAACCTGCTCGCCCGCGTCGAGCAGGGCCTATGCGACGTTCTCGGCGGCGATG

CATCCTACGGGCACCGGATCACAAAGAACCCGCTCAGCACCGCCCATGCGACCCT

CTGGGGCCCCGCAGACGCGCTCTACGAGCTGCGCGCCCTCGCACACACCCTCGA

CGAGATCCACGCACTGCCGGAGGCAGGGAACCCGCGTCGCAACGTCACCCGATC

AACGGTCGGCCGCAACGTCACCCTGTTCGACACCACCCGCATGTGGGGCATACCG

GGCCGTCCGGCACTCCTGGGGCGGCCCGGTCGCCGAATGGGAGCACACCGTATT

CGAGCACATCCACCTACTGAACGAaACGATCATCGCCGACGAgTTCGCCACAGGC

CCCCTCGGCTTGAACGAACTTAAGCACTTATCTCGATCCATTTCCCGATGGGTCTG

GCGCAACTTCACCCCCGAAACCTTCCGCGCACGCCAGAAAGCGATCAGCCTCCGT

GGAGCATCCAAAGGCGGCAAAGAAGGCGGCCACAAAGGCGGCATTGCCAGTGGC

GCATCACGGCGtGCCCATACCCGTCAACAGTTCTTGGAGGGTCTgTCATGACCACA

CGTGAACGcCTCCCCCGCAACGGCTACAGCATCGCCGCcGCTGCGAAAAGCTCG

GTGTCTCCGAGTCCACCGTCAAGCGGTGGACTTCCGAGCCACGCGAGGAGTTCGT
GGCCCGCGTTGCCGCACGCCACGCGCGGATTCGTGAGCTgCGCTCGGAGGGTCA
GAGCATGCGTGCGATTGCTGCCGAGGTCGGGGTTTCCGTGGGCACCGTGCACTA
CGCGCTGAACAAGAATCGAACTGACGCATGACCGTAACGCCGCACGATGAGCATT
TTCTTGATCGTGCACCGCTTGGCACTACGTTCGCGTGCGGTTGCACAGTGCGCGC
CACGTTCTTATCCTGCGGCCATTGTGGCTACAGCCAATGGGGGGCATCAGCAACG
GACGTTGAACCCGGTGGGCAAGTGTTACTCAGGGGGACATGCCCAGTCTGCGGC
GCTCGGATTGACGGTATGGCAGTCGTGCATGCGGCCCCACCGTCAAACTCATTCA
GGTATCAGTGAGAACCCTCATGGCACCCCTCGTGACACGTTCTCGTTGCGATCA
GCTGCGGCCAAGCTGATCTTCGGGCAGCGTTGGGTCCTGGCCACGGGTGCGCAT
GATCGTGCTCCTGTCGTTGAGGACCCGGCTAGGCTGGCGGGGTTGCCTTACtgatca
GGCCGCGTTGCTGGCGTTTTTCCATAGGCTCCGCCCCCCTGACGAGCATCACAAA
AATCGACGCTCAAGTCAGAGGTGGCGAAACCCGACAGGACTATAAAGATACCAGG
CGTTTCCCCCTGGAAGCTCCCTCGTGCGCTCTCCTGTTCCGACCCTGCCGCTTAC
CGGATACCTGTCCGCCTTTCTCCCTTCGGGAAGCGTGGCGCTTTCTCATAGCTCAC
GCTGTAGGTATCTCAGTTCGGTGTAGGTCGTTCGCTCCAAGCTGGGCTGTGTGCA
CGAACCCCCCGTTCAGCCCGACCGCTGCGCCTTATCCGGTAACTATCGTCTTGAG
TCCAACCCGGTAAGACACGACTTATCGCCACTGGCAGCAGCCACTGGTAACAGGA
TTAGCAGAGCGAGGTATGTAGGCGGTGCTACAGAGTTCTTGAAGTGGTGGCCTAA
CTACGGCTACACTAGAAGgACAGTATTTGGTATCTGCGCTCTGCTGAAGCCAGTTA
CCTTCGGAAAAAGAGTTGGTAGCTCTTGATCCGGCAAACAAACCACCGCTGGTAG
CGGTGGTTTTTTGTTTGCAAGCAGCAGATTACGCGCAGAAAAAAGGATCTCAAG
AAGATCCTTTGATCTTTTCTACGGGGTCTGACGCTCAGTGGAACGAAAACTCACGT
TAAGGGATTTTGGTCATGAGGCGCCTCACCAaTGCTTGATgAGGGAGGCACCGATC
TCGGCGATCTGACGGTTGCGCTCATCCATCGTGGCCTGGGAGCCGGTCGTGTAGA
TCACCACGATGCGGCTGGGCTTACCGTCgGGACCgAGAGCCGCGATGATGCCACG
CGAGCCGCGCTCACCAGCACCCGACTTGTCCGCGATGAACCAACCGGCCGGGAG
CGCGCTGCGCAACAGCGGGCCAGCGACCTTGTCGGCCTCCATCCAGTCGATCAG
TTGCTGACGGGAGGCCAGAGTGAGGAGTTCACCGGTCAGGAGTTTACGGAGGGT
GGTAGCCATAGCCGCGGGCATAGTCGTGTCGCGCTCGTCgTTcGGgATAGCCTCAT
TAAGTTCGGGTTCCCAACGGTCCAGACGGGTCACGTGGTCGCCCATGTTGTGGAG

AAAAGCCGTCAGCTCTTTCGGGCCACCGATGGTCGTCAACAGCAGATTGGCGGCA
GTGTTGTCGGACATAGTGATGGCCGCGGAGCACAGCTCGCGCACGGTCATCCCAT
CGGTCAGATGTTTCTCCGTGACCGGGGAGTATTCGACCAGGTCGTTCTGGGAGTA
ATGAATGCGACGGCCCAGTTGCTCCTGCCCAGCATCCACCCGGCTAAGCACAGCG
CCGCAGAGGAGGACCTTGAACGTGGACATCATGGGGAAACGCTCCTCAGGGCGG
AAAGACTCCAGAATCTTACCGGAGTTCAGGTCCAACTCGATATAACCGACGCGCG
CCCCCAGCTGATCCTCGGCGTCCTTGACCTTGACCAGGGTtTCCGGATGGGCAAA
AACCGGCAAGCAAAAGGCCGCAAAAAATGGGATAAGGGCAACGCGGAAGTGCTG
GATcGACATACTCTTCCTTTTTCAATATTATTGAAGCATTTATCAGGGTTATTGTCTC
ATGAGCGGATACATATTTGAATGTATTTAGAAAAATAAACAAATAGGGGTTCCGCGa
ctagtTCCCCGAAAAGTGCCACCTGACGTTAAGAAACCATTATTATCATGACATTAAC
CTATAAAAATAGGCGTATCACGAGGCCCTTTCGTCTaCAATCAGCGCTGACCCGGA
CCGCCACGACGGCCTGGACGACCGCCGGTACGACCACCACCCGAAGGGCCACG
ACGACCACCCTGGTCGCCCTGCTCGCGCTCCCCGGTACGACCATCGTCGCGATTA
CGGCCAGAGGACCGACGGTCACCGTGGCCGGAGGCCTGACGGTCGCGACGGTC
CTGACCACCCGTCCGACGGTCGCCACGATCCTGGCCACCCGTACGGCGGTCACC
ACGATCCTGACCACCCGTACGACGGCCGCGCTGCGGTTGGCCACCGCGCGGCTC
GTTCTTCTGGTCAAGGCGCTCGAAGAGCCGCAGCCATTGCTCCGGCCGGACGTAG
GCCACGACAGCACCACCACCCACGCCGGCATGGTCCAGCGCAGCCTCCACGCGa
CGaCGaGGGTACTTGCGCAGGAGGGAGGCTTGAATGTTGCCGCCGACACCAGTAA
AACACAACTCCACCATGCTCTCGTAGCGCTCAAGGGCAGCACCCTCCAACAGCGG
TTCAGCGCGGCGACGCAGACGCATGATGGCGGAATCGACCTTCGGGACCGGCTT
GAACAAGCGGCGATCGACCTTCTCGACGAACTCCCACTCGAAGAGCGGCCAAGTC
ATGACCGTGAGGCGGGACCAGCGGCCATAATCACCGGTGCGCTTACGGGCAAAT
TCGAGTTGGGTAACCATCGTGGCGGTtTCGATCGTCGGAGCCTCCAGGCACCAAT
CGACGATCGCAGACGTGATGCCGTACGGGATCGCGCCGACGAAGGCAAACGGCT
CGGGAGGGGGCTCGGCGGTCAGGAAGTCCGCGTTGACGACCTCGATATTCGGGT
GGCCGGACAGCTTCTCACGCAGGGACTTCGCCAAGCGCGGGTCGATCTCGTAGC
TCGTCACCTGGCGGGCCCGATCGGCAAGCTCACGGGTCAGCAGACCCTCGCCTG
GGCCAGCCTCCAGGACCGGAAGGTCGGGGCGCAGTTCGGCCGTTTCCGCGATGC
GGGCGATCGTCTTACGGTCACGAAGGAAATTCTGACCgAAtGaCGGCGGTTGCGG

TCCCGCTCAGTACGGCCCAGGACCGGGCGGTTCTGGTTCGGGTGCTGGCGGTCC
TGGTTGCGGCGACGCGGGCGCGGTTGCTCGTCGGACGACGACATGACGATCTCC
CGATGTGTCGTACGTATGGGAATTGACGAAAATCATATAGATTTTCAGTTGAAGTGT
AGCACTGCATAGTGTCATCGAAGTGTCGGCGGCAACGGTATGCCCGGTAGGGGG
TGGGTCTGCCTTTCGGTTTCGTGAACCTGATGCGGCGACGCGCATGTAGCCGATA
ACCCCTAGACTCGCCCGTGCAGATCGTCGGTGT

A second aspect of the invention refers to the composition as defined in the first aspect of the invention or in any of its preferred embodiments, for use in therapy.

A third aspect of the invention refers to a pharmaceutical composition comprising the composition as defined in the first aspect of the invention or in any of its preferred embodiments, optionally further comprising pharmaceutically acceptable excipients and/or carriers.

A fourth aspect of the invention refers to the composition as defined in the first or third aspect of the invention or in any of its preferred embodiments, for use in delivering the heterologous protein in a subject, preferably a human subject, in need thereof. Preferably for use as an antioxidant-producing strain able to rescue keratinocytes from oxidative stress in a subject, preferably a human subject, in need thereof.

A fifth aspect of the invention refers to a non-therapeutic composition, such as a cosmetic composition, comprising the composition as defined in as defined in the first aspect of the invention or in any of its preferred embodiments.

A sixth aspect of the invention refers to the use of the composition of the fifth aspect of the invention for delivering the heterologous protein in a subject, preferably a human subject, in need thereof.

## EXAMPLES

Methods

Strains and general growth conditions

*C. acnes* KPA171202 (DSM16379) was obtained from the German Collection of Microorganisms and Cell Cultures (GmbH). All C. *acnes* strains were grown in Brucella agar plates (#1012, Condalab) at 37°C in anaerobic conditions generated either with the GasPak EZ anaerobe pouch system (#BD260683, BD) or the AnaeroGen System (AN0025A, Thermo Scientific). For liquid cultures, a starting culture of 0.1 $OD_{600}$ was inoculated in brain-heart infusion (BHI) media (#1400, Condalab) and grown at 37°C 110 r.p.m. in anaerobic conditions. When appropriate, plates or liquid cultures were supplemented with either 5 $\mu$g mL$^{-1}$ chloramphenicol or 10 or 50 $\mu$g mL$^{-1}$ erythromycin. For measuring promoter and RBS strength in stationary phase, cultures were grown for 4 days. For the synthetic sensors, liquid cultures were incubated in the presence (or absence) of 100 ng mL-1 aTC or 5 $\mu$M DAPG for 3.5 or 5 days, respectively.

For the experiments involving auxotrophies, *C. acnes* was grown using defined synthetic media based on DMEM F-12 medium lacking amino acids and glucose (D9807-02A, US Biological) supplemented with 3.151g/L glucose and 1.2g/L sodium bicarbonate. The pH was adjusted to 6 with HCl and the media sterilized with a 0.2 $\mu$m filter. All amino acids were autoclaved and added unless indicated. *C. acnes* cultures were inoculated as previously mentioned with a starting $OD_{600}$ of 0.1. and grown in anaerobic conditions. Agarose 1.5% was added to the media when grown in solid in plates. To evaluate the auxotrophies, all aminoacids were added to the basal media except for the one being tested. For instance, to evaluate histidine auxotrophy, the basal medium was supplemented with all amino acids except histidine and growth was measured both in the absence and presence of histidine.

For plasmid curation, cells were passaged twice by replating them in Brucella agar plates without the selecting antibiotic. Cells were then resuspended in BHI and serial dilutions were performed in order to obtain single colonies after plating. Plates with individual colonies were replica-plated in a Brucella agar plate with the appropriate antibiotic to identify colonies that had lost the plasmid.

Plasmids and molecular biology

All plasmids in this study were based on the *Propionibacterium freudenreichii* replicative vector pBRESP36A[32,33] or on a pUC-19 based suicide vector for homologous recombination[59]. PCR fragments were usually amplified with either KAPA HiFi (KK2601, Roche) or Phanta Max (#001, Vazyme) and purified using QIAquick PCR purification or gel extraction kits (#28104/#28704, Qiagen). Plasmids were built using a modular Gibson-based cloning method34. The method consists of two steps: step 1 relies on Gibson assembly of transcriptional units into individual intermediate plasmids; in step 2, these plasmids are digested, which generates flanking regions containing overlaps that drive a second Gibson assembly, thus yielding the final construct. For step 1, all DNA parts were flanked by the same Prefix (CAGCCTGCGGTCCGG) and Suffix (TCGCTGGGACGCCCG) sequences[34]. Basically, For and Rev primers annealing to Prefix and Suffix sequences, respectively, were used for PCRs that added unique linkers to the DNA parts (Table S2, Cloning primers). PCR amplifications were column-purified as specified above and assembled using a custom Gibson enzyme mix (Centre de Regulación Genomica CRG, 1 h 50 °C) into backbones previously digested with the appropriate restriction enzymes (NEB, 1 h 37 °C) to generate the intermediate plasmids. In step 2, these intermediate plasmids were digested with enzyme combinations that produced overlapping ends, purified, and assembled as described above to yield the final constructs. Plasmids were transformed into chemically competent *E. coli* DH5$\alpha$ (MB00402, NZYtech) and plated in LB agar plates with the appropriate antibiotic (50 $\mu$g mL$^{-1}$ Ampicillin, 50 $\mu$g mL$^{-1}$ Kanamycin, 50 $\mu$g mL$^{-1}$ Spectinomycin or 25 $\mu$g mL$^{-1}$ Chloramphenicol). Liquid cultures were made in LB and incubated at 37°C 220 r.p.m. for plasmid extraction using the NZYMiniprep kit (MB01008, NZYtech). *C. acnes* plasmids were first shuttled through a $\Delta dam\ \Delta dcm\ \Delta hsdMS$ *E. coli* strain harbouring the *C. acnes IIIB methylase* for avoiding R-M system degradation upon transformation. Plasmids shuttled through this methylation strain were extracted either through the NZYMiniprep kit, the Miraprep protocol[60] or the PureLink™ Maxiprep kit (K210017, Invitrogen) to obtain enough DNA mass for electroporation. When necessary, plasmids were washed with the Amicon® ultra 0.5mL 30K centrifugal filters (UFC503096, Millipore) according to manufacturer's instructions to avoid arcing during the electroporation.

*C. acnes* electrocompetent cell preparation and transformation

*C. acnes* electrocompetent cells were prepared as previously described[28]. Briefly, *C. acnes* starting inoculum of 0.1 OD$_{600}$ were grown for 24h until ~1 OD$_{600}$. Then, cells were treated with 0.4M Sucrose and 10 $\mu$g mL$^{-1}$ penicillin G for 5h. Afterwards, cells were centrifuged at 2367 r.c.f. for 10 minutes at 4°C and resuspended in electroporation buffer consisting of 272 mM sucrose, following a second centrifugation with the same conditions. Cells were resuspended in 1 mL and transferred to a 1.5 mL microcentrifuge tube where they were washed at 1657 r.c.f. for 10 minutes followed by 4 washes at 9391 r.c.f. for 1 minute each. After the last wash, cells were resuspended in electroporation buffer supplemented with 10% glycerol, washed once again at 9391 r.c.f. for 1 minute and resuspended in 50ul of electroporation buffer with 10% glycerol per 50 mL of starting culture volume. Finally, cells were frozen in liquid nitrogen and stored at -75°C for later use. For transformation, a cell aliquot was slowly thawed on ice, resuspended in 1 mL electroporation buffer and centrifuged at 9391 r.c.f. for 1 minute. After resuspension in the same initial volume with electroporation buffer, cells were diluted four times with electroporation buffer to a final volume of 40ul and either 1000ng of DNA were added for replicative plasmid transformation or 8000ng for the suicide vectors. For electroporation, the mixture was transferred to a precooled 1 mm electroporation cuvette (#1652089, Biorad) and electroporated at 1.5 kV, 25 $\mu$F and 400 $\Omega$. Immediately after the pulse, cells were resuspended in 100 $\mu$L of BHI medium and plated on Brucella agar plates for 24h recovery at 37°C in anaerobic conditions. Next day, cells were resuspended in 1 mL BHI, centrifuged at 1657 r.c.f. for 5 minutes, resuspended in 100 $\mu$L BHI and plated in 2 Brucella agar plates supplemented with the appropriate antibiotic for a 6-day incubation in anaerobic conditions at 37°C.

Flow cytometry

*C. acnes* cells were analysed by flow cytometry on a LSR Fortessa 4L analyzer (BD) with a 488nm laser and 530/30 band pass filter for sfGFP, mCitrine and pFAST-Lime, a 405nm laser with a 450/50 band pass filter for Cerulean, and a 561nm laser with either a 610/20 band pass filter for EforCP and mCherry or a 585/15 for mKO2. Cells were centrifuged at 1657 r.c.f for 5 minutes and resuspended in PBS to ensure separation of cell events. FSC-H and SSC-H thresholds were set to exclude background events. Data was analysed using either FlowJo software (Treestar) or a custom R script using the *flowCore* package[61]. The fluorescence median of each gated population was calculated and reported in this publication as the fluorescence value of a sample in arbitrary units (a.u.).

Immunodetection of secreted SOD

Liquid cultures of recombinant C. *acnes* were grown in selective media for two days. Bacteria were collected by centrifugation and the pellet (cell fraction, C) was resuspended in Bolt LDS Sample buffer (Thermo Fisher Scientific) containing 5% beta-mercaptoethanol. The supernatant (SN) containing the secreted SOD was subjected to trichloroacetic acid (TCA) precipitation: samples were precipitated in 10% trichloroacetic acid for 30 min on ice, and precipitates were pelleted by centrifugation at ~10,000g for 15 min at 4 °C. The pellets were washed twice with 500ul cold (-20 °C) acetone, air-dried, and resuspended in Bolt LDS Sample buffer with 5% beta-mercaptoethanol. Cell and supernatant samples were boiled at 98°C for 10 min, and equal amounts of C and SN fractions were analysed using SDS-PAGE and His-tag immunodetection. Briefly, a NuPAGE 4-12% gel (Thermo Fisher Scientific) was run at 120 V for 1 h 45 min, and proteins were transferred onto a PVDF membrane (Immobilon-P Transfer Membrane, Merck Millipore) using a wet blotting apparatus running at 20 V for 1 h. Membranes were blocked overnight with 4% milk in TBST (Tris buffered saline (Bio-Rad 1706435) containing 0.05% Tween 80) and incubated for 1 h with mouse anti-His antibody (Bio-Rad MCA1396GA) diluted 1:800 in TBST-4% milk. Following three 10 min washes in TBST, membranes were incubated for 1 h in horseradish peroxidase-coupled anti-mouse antibody (SantaCruz sc516102) diluted 1:800 in TBST-4% milk, and washed again three times 10 min each in TBST. Membranes were developed with Pierce ECL Western Blotting Substrate (Thermo Fisher Scientific) and recorded using a ChemiDoc MP Imaging System (Bio-Rad).

N/TERT-2G Cell culture, UV-exposure and AD monolayer model

The immortalized keratinocyte cell line N/TERT-2G[55,56] was obtained from the J. Rheinwald laboratory (Harvard Medical School, Boston, USA). These cells were passaged in T25 or T75 flasks in keratinocyte-serum free EpiLife medium (#MEPI500CA, Gibco) with human keratinocyte culture supplement (HKGS) (#S0015, Gibco) based on previously published protocols[62] in a humidified 5% $CO_2$ incubator. Alternatively, CnT-Prime (CNT-PR, CELLnTEC) media was also used.

For UV-exposure, cells were seeded in $3.6 \times 10^4$ cells mL$^{-1}$ in 24 well plates. When confluent, cells were exposed for 30 minutes to four UV-B lamps (302 nm) (Gel Dock XR+, Biorad). Then, cells were washed with Hanks' balanced salt solution (HBSS) and dyed for 30 minutes at 37°C with 20 $\mu$M 6-chloromethyl-2',7'-dichlorodihydrofluorescein diacetate (CM-H2DCFDA) (C6827, Invitrogen). After washing again with HBSS, we applied the 0.2 $\mu$m filtered bacterial supernatant for 5h on the cells, and we measured the ROS levels using a M Nano Infitine 200 Pro plate reader (Tecan) at an excitation/emission of 485/535nm.

SOD activity quantification

0.2 $\mu$m-filtered C. acnes supernatants were transferred to a 96-well plate to measure the SOD activity using an SOD activity assay kit (#CS0009, Sigma) following manufacturer's instructions. Briefly, 20 $\mu$L from supernatants were mixed with 20 $\mu$L of the WST dye, and 160 $\mu$L of xanthine oxidase were added to start the reaction. The SOD activity was calculated using a standard calibration curve. First, the absorbance at 450nm from each sample was averaged across replicates. To obtain the linearized SOD rate, a blank control was subtracted from all samples and standards, and resulting values were divided by the absorbance of the control reaction lacking SOD. The standards were then used for a linear regression to obtain the calibration parameters as the slope and intercept. Then the following formula was applied.

$$SOD\ activity\ (units/mL) = \frac{SampleLSR\ -\ intercept}{Slope}\ x\ 10$$

Where Sample$_{LSR}$ is the linearized SOD rate of the sample and the multiplication by 10 corresponds to the dilution factor. A calibration curve was only considered valid when its R$^2$ was higher than 0.95.

RNA extraction

Total RNA was isolated by E.Z.N.A. Total RNA kit (#R6834-02, Omega bio-tek). For bacterial RNA isolation, cultures were centrifuged at 8000 r.c.f. for 5 minutes and resuspended in 1 mL TRK Lysis buffer. Then, cells were lysed using Precellys® 0.1 mm silica beads (432-3754, VWR) for 15 minutes in a Disruptor Genie (SI-D258, Scientific Industries) and the supernatant was collected after centrifugation for 4 minutes at 10.000 r.c.f. for further processing. For human RNA isolation, 350 $\mu$L of TRK lysis buffer was added directly to cells and taken for further processing according to manufacturer's instructions. Briefly, either bacterial or human RNA in TRK buffer was mixed with an equal volume of fresh ethanol 70% and loaded in the RNA isolation columns by centrifugation at 13.000 r.c.f. for 1 minute. Then columns were washed with 500 $\mu$L of Wash Buffer I, 500 $\mu$L of Wash Buffer II twice and a last 2 minute centrifugation to let the column dry. The RNA was eluted with 40 $\mu$L RNase free water and concentration was measured with NanoDrop® One Spectrophotometer (ND-ONE-W, ThermoFisher)

RNA-seq

Isolated RNA was analyzed for purity and integrity using Bioanalyzer (Agilent Technologies GmbH, Germany) following library construction and RNA-sequencing by Macrogen Inc. (Seoul, South Korea) using the Truseq Stranded Total RNA and sequenced using an Illumina at 60M pair-reads depth. RNA-seq analysis was performed using the nf-core RNA-seq pipeline v3.0[63,64] in Nextflow v20.12.0-edge[65]. Raw paired-end reads were trimmed using Trim Galore v0.6.6 and aligned to the C. acnes KPA171202 reference genome (Genbank AE017283) using STAR v2.6.1d[66] and SAMtools v1.10[67].

Quality control was performed using FastQC v0.11.9. Mapped reads were counted using mpileup from BCFtools in htslib v1.1[68]. For temperature-sensitive promoters, differential gene expression analysis between three heat shock samples and three controls was performed on the normalized read counts using DeSeq2[69]. Genes with a log fold-change greater than 3 were selected for further manual inspection of the read coverage across the genetic locus. We extracted either 200bp upstream the start codon or the whole intergenic region for those genes whose upregulation was consistent all over their ORF. Similarly, constitutive promoters were selected among the genes within the 15% lowest variation coefficient (CV) and after manual inspection of the read coverage for uniformity. Again, the regulatory sequence was identified as either the intergenic region or 200bp upstream from the start codon. For endogenous terminator sequences, mean change points were detected across the coverage pileup using the changepoint R package cpt.mean. Then, we calculated the segments between change points and selected those segments containing at least one stop codon that had the greatest drop in expression compared with their neighbouring segments. After manual inspection of those segments fulfilling these criteria, we selected as terminator sequences the intergenic regions within the selected segments.

RT-qPCR

500ng of isolated total RNA were treated with DNasel (18068015, Invitrogen) and used for cDNA synthesis using the RevertAid First Strand cDNA Synthesis kit (K1622, ThermoFisher) according to the manufacturer's protocols. Subsequent real-time quantitative PCR (RT-qPCR) was performed using PowerUp™ SYBR™ Green Master Mix (A25742, Life Technologies). Target gene expression levels were normalized using the housekeeping gene gyrase B (GyrB) from *C. acnes*. The $\Delta\Delta CT$ method was used to calculate relative mRNA expression levels[70].

Statistics and reproducibility

Statistical analysis was performed using R and RStudio. Each experiment includes at least three independent replicates and graphs include the mean $\pm$ s.d. Unpaired one-way analysis of variance (ANOVA) was used for comparison between multiple groups followed by Tukey's multiple comparison post hoc test. Results with p-value < 0.001 were considered highly statistically significant (\*\*\*). Results with p-value < 0.05 were considered as significant (\*). All blots and gels were repeated at least two times.

Terminator efficiency was calculated with the following equation as previously described.

$$\text{Terminator efficiency} = 1 - \left( \frac{\text{mCherry Terminator}}{\text{mCherry Spacer\_1}} \right) \Big/ \left( \frac{\text{sfGFP Terminator}}{\text{sfGFP Spacer\_1}} \right)$$

Where Spacer_1 corresponds to the mCherry or the sfGFP fluorescence levels from the control harbouring only the Spacer_1 sequence and no terminator, and Terminator corresponds to the fluorescence levels of the terminator evaluated. Error bars were calculated with the following formula, propagating the error of three biological replicates:

$$\text{Propagated error} = \sqrt{\left( \frac{\sigma_{\text{mCherry Ratio}}}{\text{GFP Ratio}} \right)^2 + \left( \frac{\text{mCherry Ratio} \cdot \sigma_{\text{GFP Ratio}}}{\text{GFP Ratio}^2} \right)^2}$$

References

1. Hay, R. J., Augustin, M., Griffiths, C. E. M., Sterry, W. & Board of the International League of Dermatological Societies and the Grand Challenges Consultation groups. The global challenge for skin health. Br. J. Dermatol. 172, 1469-1472 (2015).
2. Hay, R. J. et al. The global burden of skin disease in 2010: an analysis of the prevalence and impact of skin conditions. J. Invest. Dermatol. 134, 1527-1534 (2014).
3. Richard, M. A. et al. Prevalence of most common skin diseases in Europe: a population-based study. J. Eur. Acad. Dermatol. Venereol. 36, 1088-1096 (2022).
4. Quan, T. Molecular insights of human skin epidermal and dermal aging. J. Dermatol. Sci. 112, 48-53 (2023).
5. Nikam, R. V., Gowtham, M., More, P. S. & Shinde, A. S. Current and emerging prospects in the psoriatic treatment.

Int. Immunopharmacol. 120, 110331 (2023).

6. Sesi, J. & Feldman, S. R. Comparative efficacy of systemic treatments for atopic dermatitis in adults. Expert Rev. Clin. Immunol. 20, 313-320 (2024).

7. Heinz, K. C., Beaudart, C., Willems, D., Wiethoff, I. & Hiligsmann, M. Cost-Effectiveness of Emerging Treatments for Atopic Dermatitis: A Systematic Review. Pharmacoeconomics 41, 1415-1435 (2023).

8. Zimmermann, M. et al. Economic Evaluation of Dupilumab for Moderate-to-Severe Atopic Dermatitis: A Cost-Utility Analysis. J. Drugs Dermatol. 17, 750-756 (2018).

9. Isabella, V. M. et al. Development of a synthetic live bacterial therapeutic for the human metabolic disease phenylketonuria. Nat. Biotechnol. 36, 857-864 (2018).

10. Puurunen, M. K. et al. Safety and pharmacodynamics of an engineered E. coli Nissle for the treatment of phenylketonuria: a first-in-human phase 1/2a study. Nat Metab 3, 1125-1132 (2021).

11. Lubkowicz, D. et al. An engineered bacterial therapeutic lowers urinary oxalate in preclinical models and in silico simulations of enteric hyperoxaluria. Mol. Syst. Biol. 18, e10539 (2022).

12. Mao, N., Cubillos-Ruiz, A., Cameron, D. E. & Collins, J. J. Probiotic strains detect and suppress cholera in mice. Sci. Transl. Med. 10, (2018).

13. Leventhal, D. S. et al. Immunotherapy with engineered bacteria by targeting the STING pathway for anti-tumor immunity. Nat. Commun. 11, 2739 (2020).

14. Mazzolini, R. et al. Engineered live bacteria suppress Pseudomonas aeruginosa infection in mouse lung and dissolve endotracheal-tube biofilms. Nat. Biotechnol. 41, 1089-1098 (2023).

15. Montero-Blay, A. et al. Bacterial expression of a designed single-chain IL-10 prevents severe lung inflammation. Mol. Syst. Biol. 19, e11037 (2023).

16. Öhnstedt, E. et al. Engineered bacteria to accelerate wound healing: an adaptive, randomised, double-blind, placebo-controlled, first-in-human phase 1 trial. EClinicalMedicine 60, 102014 (2023).

17. Chen, Y. E. et al. Engineered skin bacteria induce antitumor T cell responses against melanoma. Science 380, 203-210 (2023).

18. Liu, F. et al. Engineered Skin Microbiome Reduces Mosquito Attraction to Mice. bioRxiv (2023) doi:10.1101/2023.12.20.572663.

19. Oh, J. et al. Temporal Stability of the Human Skin Microbiome. Cell 165, 854-866 (2016).

20. Zhou, W. et al. Host-Specific Evolutionary and Transmission Dynamics Shape the Functional Diversification of Staphylococcus epidermidis in Human Skin. Cell 180, 454-470.e18 (2020).

21. Oh, J. et al. Biogeography and individuality shape function in the human skin metagenome. Nature 514, 59-64 (2014).

22. Gribbon, E. M., Cunliffe, W. J. & Holland, K. T. Interaction of Propionibacterium acnes with skin lipids in vitro. J. Gen. Microbiol. 139, 1745-1751 (1993).

23. Barry, B. W. Drug delivery routes in skin: a novel approach. Adv. Drug Deliv. Rev. 54 Suppl 1, S31-40 (2002).

24. Conwill, A. et al. Anatomy promotes neutral coexistence of strains in the human skin microbiome. Cell Host Microbe 30, 171-182.e7 (2022).

25. Scholz, C. F. P., Brüggemann, H., Lomholt, H. B., Tettelin, H. & Kilian, M. Genome stability of Propionibacterium acnes: a comprehensive study of indels and homopolymeric tracts. Sci. Rep. 6, 20662 (2016).

26. Moe-Behrens, G. H. G., Davis, R. & Haynes, K. A. Preparing synthetic biology for the world. Front. Microbiol. 4, 5 (2013).

27. Paetzold, B. et al. Skin microbiome modulation induced by probiotic solutions. Microbiome 7, 1-9 (2019).

28. Knödlseder, N. et al. Delivery of a sebum modulator by an engineered skin microbe in mice. Nat. Biotechnol. (2024) doi:10.1038/s41587-023-02072-4.

29. Salis, H. M., Mirsky, E. A. & Voigt, C. A. Automated design of synthetic ribosome binding sites to control protein expression. Nat. Biotechnol. 27, 946-950 (2009).

30. Scholz, C. F. P., Jensen, A., Lomholt, H. B., Brüggemann, H. & Kilian, M. A novel high-resolution single locus sequence typing scheme for mixed populations of Propionibacterium acnes in vivo. PLoS One 9, e104199 (2014).

31. Kilian, M., Scholz, C. F. P. & Lomholt, H. B. Multilocus sequence typing and phylogenetic analysis of Propionibacterium acnes. J. Clin. Microbiol. 50, 1158-1165 (2012).

32. Lood, R. Propionibacterium acnes and its phages. (Lund University, 2011).

33. Jore, J. P., van Luijk, N., Luiten, R. G., van der Werf, M. J. & Pouwels, P. H. Efficient transformation system for Propionibacterium freudenreichii based on a novel vector. Appl. Environ. Microbiol. 67, 499-503 (2001).

34. Santos-Moreno, J. & Schaerli, Y. A Framework for the Modular and Combinatorial Assembly of Synthetic Gene Circuits. ACS Synth. Biol. 8, 1691-1697 (2019).

35. Lin, Y.-F., A, D. R., Guan, S., Mamanova, L. & McDowall, K. J. A combination of improved differential and global RNA-seq reveals pervasive transcription initiation and events in all stages of the life-cycle of functional RNAs in Propionibacterium acnes, a major contributor to wide-spread human disease. BMC Genomics 14, 620 (2013).

36. Benaissa, H. et al. Engineering of a fluorescent chemogenetic reporter with tunable color for advanced live-cell imaging. Nat. Commun. 12, 6989 (2021).

37. Pinilla-Redondo, R., Riber, L. & Sorensen, S. J. Fluorescence Recovery Allows the Implementation of a Fluorescence Reporter Gene Platform Applicable for the Detection and Quantification of Horizontal Gene Transfer in Anoxic Environments. Appl. Environ. Microbiol. 84, (2018).

38. Ransom, E. M., Ellermeier, C. D. & Weiss, D. S. Use of mCherry Red fluorescent protein for studies of protein localization and gene expression in Clostridium difficile. Appl. Environ. Microbiol. 81, 1652-1660 (2015).

39. Chen, Y.-J. et al. Characterization of 582 natural and synthetic terminators and quantification of their design constraints. Nat. Methods 10, 659-664 (2013).

40. Baba, T. et al. Construction of Escherichia coli K-12 in-frame, single-gene knockout mutants: the Keio collection. Mol. Syst. Biol. 2, 2006.0008 (2006).

41. Nielsen, P. A. Role of reduced sulfur compounds in nutrition of Propionibacterium acnes. J. Clin. Microbiol. 17, 276-279 (1983).

42. Mark, H. & Harding, C. R. Amino acid composition, including key derivatives of eccrine sweat: potential biomarkers of certain atopic skin conditions. Int. J. Cosmet. Sci. 35, 163-168 (2013).

43. Kim, N. M., Sinnott, R. W. & Sandoval, N. R. Transcription factor-based biosensors and inducible systems in non-model bacteria: current progress and future directions. Curr. Opin. Biotechnol. 64, 39-46 (2020).

44. Xiao, Y., Jiang, W. & Zhang, F. Developing a Genetically Encoded, Cross-Species Biosensor for Detecting Ammonium and Regulating Biosynthesis of Cyanophycin. ACS Synth. Biol. 6, 1807-1815 (2017).

45. Immethun, C. M. et al. Oxygen-responsive genetic circuits constructed in Synechocystis sp. PCC 6803. Biotechnol. Bioeng. 113, 433-442 (2016).

46. DeLorenzo, D. M., Henson, W. R. & Moon, T. S. Development of Chemical and Metabolite Sensors for Rhodococcus opacus PD630. ACS Synth. Biol. 6, 1973-1978 (2017).

47. Rottinghaus, A. G., Ferreiro, A., Fishbein, S. R. S., Dantas, G. & Moon, T. S. Genetically stable CRISPR-based kill switches for engineered microbes. Nat. Commun. 13, 672 (2022).

48. Abedi, M. H. et al. Ultrasound-controllable engineered bacteria for cancer immunotherapy. Nat. Commun. 13, 1585 (2022).

49. Das Gupta, T., Bandyopadhyay, B. & Das Gupta, S. K. Modulation of DNA-binding activity of Mycobacterium tuberculosis HspR by chaperones. Microbiology 154, 484-490 (2008).

50. Spohn, G. & Scarlato, V. The autoregulatory HspR repressor protein governs chaperone gene transcription in Helicobacter pylori. Mol. Microbiol. 34, 663-674 (1999).

51. Georgi, C., Buerger, J., Hillen, W. & Berens, C. Promoter strength driving TetR determines the regulatory properties of Tet-controlled expression systems. PLoS One 7, e41620 (2012).

52. D'Orazio, M. et al. Regulatory and structural properties differentiating the chromosomal and the bacteriophage-associated Escherichia coli O157:H7 Cu, Zn superoxide dismutases. BMC Microbiol. 8, 166 (2008).

53. Holland, C. et al. Proteomic identification of secreted proteins of Propionibacterium acnes. BMC Microbiol. 10, 230 (2010).

54. Allhorn, M., Arve, S., Brüggemann, H. & Lood, R. A novel enzyme with antioxidant capacity produced by the ubiquitous skin colonizer Propionibacterium acnes. Sci. Rep. 6, 36412 (2016).

55. Smits, J. P. H. et al. Immortalized N/TERT keratinocytes as an alternative cell source in 3D human epidermal models. Sci. Rep. 7, 11838 (2017).

56. Dickson, M. A. et al. Human keratinocytes that express hTERT and also bypass a p16(INK4a)-enforced mechanism that limits life span become immortal yet retain normal growth and differentiation characteristics. Mol. Cell. Biol. 20, 1436-1447 (2000).

57. Müller, I. E. et al. Gene networks that compensate for crosstalk with crosstalk. Nat. Commun. 10, 4028 (2019).

58. Inda-Webb, M. E. et al. Sub-1.4 cm3 capsule for detecting labile inflammatory biomarkers in situ. Nature 620, 386-392 (2023).

59. Sörensen, M. et al. Mutagenesis of Propionibacterium acnes and analysis of two CAMP factor knock-out mutants. J. Microbiol. Methods 83, 211-216 (2010).

60. Pronobis, M. I., Deuitch, N. & Peifer, M. The Miraprep: A Protocol that Uses a Miniprep Kit and Provides Maxiprep Yields. PLoS One 11, e0160509 (2016).

61. Hahne, F. et al. flowCore: a Bioconductor package for high throughput flow cytometry. BMC Bioinformatics 10, 106 (2009).

62. Rikken, G., Niehues, H. & van den Bogaard, E. H. Organotypic 3D Skin Models: Human Epidermal Equivalent Cultures from Primary Keratinocytes and Immortalized Keratinocyte Cell Lines. in Molecular Dermatology: Methods and Protocols (eds. Botchkareva, natalia V. & Westgate, G. E.) 45-61 (Springer US, New York, NY, 2020).

63. Ewels, P. A. et al. The nf-core framework for community-curated bioinformatics pipelines. Nat. Biotechnol. 38, 276-278 (2020).

64. Patel, H. et al. Nf-Corelrnaseq: Nf-Core/rnaseq v3.0 - Silver Shark. (Zenodo, 2020). doi:10.5281/ZENODO.4323183.

65. Di Tommaso, P. et al. Nextflow enables reproducible computational workflows. Nat. Biotechnol. 35, 316-319 (2017).

66. Dobin, A. et al. STAR: ultrafast universal RNA-seq aligner. Bioinformatics 29, 15-21 (2013).

67. Li, H. et al. The Sequence Alignment/Map format and SAMtools. Bioinformatics 25, 2078-2079 (2009).

68. Li, H. A statistical framework for SNP calling, mutation discovery, association mapping and population genetical parameter estimation from sequencing data. Bioinformatics 27, 2987-2993 (2011).

69. Love, M. I., Huber, W. & Anders, S. Moderated estimation of fold change and dispersion for RNA-seq data with DESeq2. Genome Biol. 15, 550 (2014).

70. Livak, K. J. & Schmittgen, T. D. Analysis of relative gene expression data using real-time quantitative PCR and the 2(-Delta Delta C(T)) Method. Methods 25, 402-408 (2001).

## Claims

1. A composition comprising *Cutibacterium acnes (C. acnes)* bacterial strains, wherein said strains are **characterized by** being modified to secrete a heterologous protein by insertion of an expression construct comprising at least one or more transcription promoter sequences, one or more ORFs (Open Reading Frames) encoding the heterologous protein, and optionally one or more transcription termination sequences; wherein the heterologous protein is **characterized by** comprising at the N-terminus of the protein sequence, a secretion signal which is processed by *C. acnes* to secrete the protein, and wherein the heterologous protein is the superoxide dismutase enzyme.

2. The composition according to claim 1, wherein the secretion signal is of SEQ ID No. 1 or a functional variant thereof that has at least 90%, 91%, 92%, 93%, 94%, 95%, 96%, 97%, 98%, or 99% sequence identity with the full/length sequence of SEQ ID NO 1 with the proviso that said functional variant can be processed by *C. acnes* cells to secrete the heterologous protein.

3. The composition according to any one of claims 1 or 2, wherein the superoxide dismutase enzyme is the SodC-F1 from the enterohemorrhagic E. coli O157:H7 of SEQ ID NO 2 or any sequence that has at least 80%, 90%, 91%, 92%, 93%, 94%, 95%, 96%, 97%, 98%, or 99% identity with the full/length sequence of SEQ ID No. 2 that when secreted in one or multiple copies by using the secretion signal of SEQ ID NO 1 is capable of reducing ROS levels.

4. The composition according to any one of claims 1 to 3, wherein the superoxide dismutase enzyme is the SodC-F1 from the enterohemorrhagic E. coli O157:H7 of SEQ ID NO 2 and the secretion signal is of SEQ ID No. 1.

5. The composition according to any one of claims 1 to 4, wherein the one or more transcription promoter sequences is the promoter of SEQ ID NO 3, 5 or 6, 15 or the inducible promoter of any one of SEQ ID NO 18 to 22, or any sequence that has at least 80%, 90%, 91%, 92%, 93%, 94%, 95%, 96%, 97%, 98%, or 99% identity with the full/length sequence of SEQ ID No. 3, 5 or 6, or 15 or SEQ ID NO. 18 to 22, that when operably linked to the heterologous protein is capable of expressing the said protein.

6. The composition according to any one of claims 1 to 5, wherein the expression construct further comprises a ribosome binding sites (RBS): preferably the RBS is the E. *coli* RBS sequences of SEQ ID No 7 or 8 or any sequence that has at least 80%, 90%, 91%, 92%, 93%, 94%, 95%, 96%, 97%, 98%, or 99% identity with the full/length sequence of SEQ ID No. 7 or 8.

7. The composition according to any one of claims 1 or 2, wherein the expression construct is of SEQ ID NO 9, 10, 11, or 16, or any sequence that has at least 80%, 90%, 91%, 92%, 93%, 94%, 95%, 96%, 97%, 98%, or 99% identity with the full/length sequence of SEQ ID NO 9, 10, 11, or 16.

8. The composition according to any one of claims 1 to 7, wherein the expression construct is comprised in a plasmid, preferably a plasmid capable of replicating in *C. acnes* and comprising an origin of replication for *C. acnes.*

9. The composition according to any one of claims 1 to 8, wherein the strains are mutant live auxotrophic *Cutibacterium acnes* (*C. acnes*) bacterial strains, preferably for Lysine, Histidine or Proline, with the proviso that said mutant live auxotrophic *C. acnes* bacterial strains do not comprise any heterologous antibiotic resistance genes.

10. The composition according to any one of claims 1 to 9, wherein the expression construct is comprised in a plasmid and the plasmid comprising the said expression construct is selected from any one of SEQ ID NO 12, 13, 14, or 17 or any sequence that has at least 80%, 90%, 91%, 92%, 93%, 94%, 95%, 96%, 97%, 98%, or 99% identity with the full/length sequence of SEQ ID NO 12, 13, 14, or 17.

11. The composition according to any one of claims 1 to 10, for use in therapy.

12. A pharmaceutical composition comprising the composition as defined in any one of claims 1 to 10 optionally further comprising pharmaceutically acceptable excipients and/or carriers.

13. The composition according to any of claims 1 to 10 or 12, for use in delivering the heterologous protein in a subject, preferably a human subject, in need thereof, preferably for use as an antioxidant-producing strain able to rescue keratinocytes from oxidative stress in a subject, preferably a human subject, in need thereof.

14. A non-therapeutic composition, such as a cosmetic composition, comprising the composition as defined in any one of claims 1 to 10.

15. A non-therapeutic use of the composition according to claim 14 for delivering the heterologous protein in a subject, preferably a human subject, in need thereof.

**FIG. 1**

# FIG. 1 (cont.)

**FIG. 1 (cont.)**

EP 4 729 602 A1

46

## FIG. 2

**A**

CRISPRi

**FIG. 2 (cont.)**

**B**

CRISPRi screen for auxotrophy

**C**

ΔLysA
BxbI recombinase

**D**

Auxotroph strains evaluation

**FIG. 3**

# FIG. 3 (cont.)

FIG. 4

α-His          α-His

**FIG. 5**

Europäisches
Patentamt

European
Patent Office

Office européen
des brevets

## EUROPEAN SEARCH REPORT

**Application Number**

EP 24 38 3134

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ZHENG MENGLI ET AL: "The Applications and Mechanisms of Superoxide Dismutase in Medicine, Food, and Cosmetics", ANTIOXIDANTS, vol. 12, no. 9, 27 August 2023 (2023-08-27), page 1675, XP093256120, ISSN: 2076-3921, DOI: 10.3390/antiox12091675 * page 9, paragraph 2 * * page 15, paragraph 1 * * figure 5 * ----- | 1,2,5-15 | INV. C12N1/20 C12N9/02 C12P21/02 |
| Y | EP 4 215 204 A1 (UNIV POMPEU FABRA [ES]) 26 July 2023 (2023-07-26) * claims 1-4, 7, 11-13; sequences 6,13 * * paragraph [0042] * ----- | 1,2,5-15 | |
| A | EP 0 952 224 A2 (KOREA INST SCI & TECH [KR]) 27 October 1999 (1999-10-27) * paragraphs [0014], [0035], [0039]; claim 12 * ----- | 1-15 | |
| A | NARENDRA TUTEJA ET AL: "Heterologous expression and biochemical characterization of a highly active and stable chloroplastic CuZn-superoxide dismutase from Pisum sativum", BMC BIOTECHNOLOGY, BIOMED CENTRAL LTD, vol. 15, no. 1, 8 February 2015 (2015-02-08), page 3, XP021211469, ISSN: 1472-6750, DOI: 10.1186/S12896-015-0117-0 * page 3, left-hand column, paragraphs 1,2 * ----- -/-- | 1-15 | TECHNICAL FIELDS SEARCHED (IPC) C12N C12P C12R |

The present search report has been drawn up for all claims

| Place of search | Date of completion of the search | Examiner |
|---|---|---|
| The Hague | 14 March 2025 | Blanco Urgoiti, B |

page 1 of 2

Europäisches
Patentamt

European
Patent Office

Office européen
des brevets

**EUROPEAN SEARCH REPORT**

Application Number

EP 24 38 3134

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | D'ORAZIO MELANIA ET AL: "Regulatory and structural properties differentiating the chromosomal and the bacteriophage-associated Escherichia coli O157:H7 Cu, Zn Superoxide Dismutases", BMC MICROBIOLOGY, BIOMED CENTRAL LTD, GB, vol. 8, no. 1, 1 October 2008 (2008-10-01), page 166, XP021042379, ISSN: 1471-2180, DOI: 10.1186/1471-2180-8-166 * page 2, left-hand column, paragraph 2 * ----- | 1-15 | |
| | | | TECHNICAL FIELDS SEARCHED (IPC) |

The present search report has been drawn up for all claims

| Place of search | Date of completion of the search | Examiner |
|---|---|---|
| The Hague | 14 March 2025 | Blanco Urgoiti, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 38 3134

This annex lists the patent family members relating to the patent documents cited in the above-mentioned European search report.
The members are as contained in the European Patent Office EDP file on
The European Patent Office is in no way liable for these particulars which are merely given for the purpose of information.

14-03-2025

| Patent document cited in search report | | Publication date | Patent family member(s) | | Publication date |
|---|---|---|---|---|---|
| EP 4215204 | A1 | 26-07-2023 | EP | 4215204 A1 | 26-07-2023 |
| | | | EP | 4469076 A1 | 04-12-2024 |
| | | | JP | 2025504502 A | 12-02-2025 |
| | | | KR | 20240141189 A | 25-09-2024 |
| | | | WO | 2023139291 A1 | 27-07-2023 |
| EP 0952224 | A2 | 27-10-1999 | EP | 0952224 A2 | 27-10-1999 |
| | | | JP | 3307604 B2 | 24-07-2002 |
| | | | JP | H11341929 A | 14-12-1999 |
| | | | US | 6084152 A | 04-07-2000 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HAY, R. J.** ; **AUGUSTIN, M.** ; **GRIFFITHS, C. E. M.** ; **STERRY, W.** The global challenge for skin health. *Br. J. Dermatol.*, 2015, vol. 172, 1469-1472
- **HAY, R. J. et al.** The global burden of skin disease in 2010: an analysis of the prevalence and impact of skin conditions. *J. Invest. Dermatol*, 2014, vol. 134, 1527-1534
- **RICHARD, M. A. et al.** Prevalence of most common skin diseases in Europe: a population-based study. *J. Eur. Acad. Dermatol. Venereol.*, 2022, vol. 36, 1088-1096
- **QUAN, T.** Molecular insights of human skin epidermal and dermal aging. *J. Dermatol. Sci.*, 2023, vol. 112, 48-53
- **NIKAM, R. V.** ; **GOWTHAM, M.** ; **MORE, P. S.** ; **SHINDE, A. S.** Current and emerging prospects in the psoriatic treatment. *Int. Immunopharmacol*, 2023, vol. 120, 110331
- **SESI, J.** ; **FELDMAN, S. R.** Comparative efficacy of systemic treatments for atopic dermatitis in adults. *Expert Rev. Clin. Immunol.*, 2024, vol. 20, 313-320
- **HEINZ, K. C.** ; **BEAUDART, C.** ; **WILLEMS, D.** ; **WIETHOFF, I.** ; **HILIGSMANN, M.** Cost-Effectiveness of Emerging Treatments for Atopic Dermatitis: A Systematic Review. *Pharmacoeconomics*, 2023, vol. 41, 1415-1435
- **ZIMMERMANN, M. et al.** Economic Evaluation of Dupilumab for Moderate-to-Severe Atopic Dermatitis: A Cost-Utility Analysis. *J. Drugs Dermatol.*, 2018, vol. 17, 750-756
- **ISABELLA, V. M. et al.** Development of a synthetic live bacterial therapeutic for the human metabolic disease phenylketonuria. *Nat. Biotechnol.*, 2018, vol. 36, 857-864
- **PUURUNEN, M. K. et al.** Safety and pharmacodynamics of an engineered E. coli Nissle for the treatment of phenylketonuria: a first-in-human phase 1/2a study. *Nat Metab*, 2021, vol. 3, 1125-1132
- **LUBKOWICZ, D. et al.** An engineered bacterial therapeutic lowers urinary oxalate in preclinical models and in silico simulations of enteric hyperoxaluria. *Mol. Syst. Biol.*, 2022, vol. 18, e10539
- **MAO, N.** ; **CUBILLOS-RUIZ, A.** ; **CAMERON, D. E.** ; **COLLINS, J. J.** Probiotic strains detect and suppress cholera in mice. *Sci. Transl. Med.*, 2018, vol. 10

- **LEVENTHAL, D. S. et al.** Immunotherapy with engineered bacteria by targeting the STING pathway for anti-tumor immunity. *Nat. Commun.*, 2020, vol. 11, 2739
- **MAZZOLINI, R. et al.** Engineered live bacteria suppress Pseudomonas aeruginosa infection in mouse lung and dissolve endotracheal-tube biofilms. *Nat. Biotechnol.*, 2023, vol. 41, 1089-1098
- **MONTERO-BLAY, A. et al.** Bacterial expression of a designed single-chain IL-10 prevents severe lung inflammation. *Mol. Syst. Biol.*, 2023, vol. 19, e11037
- **ÖHNSTEDT, E. et al.** Engineered bacteria to accelerate wound healing: an adaptive, randomised, double-blind, placebo-controlled, first-in-human phase 1 trial. *EClinicalMedicine*, 2023, vol. 60, 102014
- **CHEN, Y. E. et al.** Engineered skin bacteria induce antitumor T cell responses against melanoma. *Science*, 2023, vol. 380, 203-210
- **LIU, F. et al.** Engineered Skin Microbiome Reduces Mosquito Attraction to Mice. *bioRxiv*, 2023
- **OH, J. et al.** Temporal Stability of the Human Skin Microbiome. *Cell*, 2016, vol. 165, 854-866
- **ZHOU, W. et al.** Host-Specific Evolutionary and Transmission Dynamics Shape the Functional Diversification of Staphylococcus epidermidis in Human Skin. *Cell*, 2020, vol. 180, 454-470
- **OH, J. et al.** Biogeography and individuality shape function in the human skin metagenome. *Nature*, 2014, vol. 514, 59-64
- **GRIBBON, E. M.** ; **CUNLIFFE, W. J.** ; **HOLLAND, K. T.** Interaction of Propionibacterium acnes with skin lipids in vitro. *J. Gen. Microbiol.*, 1993, vol. 139, 1745-1751
- **BARRY, B. W.** Drug delivery routes in skin: a novel approach. *Adv. Drug Deliv. Rev.*, 2002, vol. 54, 31-40
- **CONWILL, A. et al.** Anatomy promotes neutral coexistence of strains in the human skin microbiome. *Cell Host Microbe*, 2022, vol. 30, 171-182
- **SCHOLZ, C. F. P.** ; **BRÜGGEMANN, H.** ; **LOMHOLT, H. B.** ; **TETTELIN, H.** ; **KILIAN, M.** Genome stability of Propionibacterium acnes: a comprehensive study of indels and homopolymeric tracts. *Sci. Rep.*, 2016, vol. 6, 20662
- **MOE-BEHRENS, G. H. G.** ; **DAVIS, R.** ; **HAYNES, K. A.** Preparing synthetic biology for the world. *Front. Microbiol.*, 2013, vol. 4, 5

- **PAETZOLD, B. et al.** Skin microbiome modulation induced by probiotic solutions. *Microbiome*, 2019, vol. 7, 1-9
- **KNÖDLSEDER, N. et al.** Delivery of a sebum modulator by an engineered skin microbe in mice. *Nat. Biotechnol.*, 2024
- **SALIS, H. M.** ; **MIRSKY, E. A.** ; **VOIGT, C. A.** Automated design of synthetic ribosome binding sites to control protein expression. *Nat. Biotechnol.*, 2009, vol. 27, 946-950
- **SCHOLZ, C. F. P.** ; **JENSEN, A.** ; **LOMHOLT, H. B.** ; **BRÜGGEMANN, H.** ; **KILIAN, M.** A novel high-resolution single locus sequence typing scheme for mixed populations of Propionibacterium acnes in vivo. *PLoS One*, 2014, vol. 9, e104199
- **KILIAN, M.** ; **SCHOLZ, C. F. P.** ; **LOMHOLT, H. B.** Multilocus sequence typing and phylogenetic analysis of Propionibacterium acnes. *J. Clin. Microbiol.*, 2012, vol. 50, 1158-1165
- **LOOD, R.** Propionibacterium acnes and its phages. Lund University, 2011
- **JORE, J. P.** ; **VAN LUIJK, N.** ; **LUITEN, R. G.** ; **VAN DER WERF, M. J.** ; **POUWELS, P. H.** Efficient transformation system for Propionibacterium freudenreichii based on a novel vector. *Appl. Environ. Microbiol.*, 2001, vol. 67, 499-503
- **SANTOS-MORENO, J.** ; **SCHAERLI, Y.** A Framework for the Modular and Combinatorial Assembly of Synthetic Gene Circuits. *ACS Synth. Biol.*, 2019, vol. 8, 1691-1697
- **LIN, Y.-F., A, D. R.** ; **GUAN, S.** ; **MAMANOVA, L.** ; **MCDOWALL, K. J.** A combination of improved differential and global RNA-seq reveals pervasive transcription initiation and events in all stages of the life-cycle of functional RNAs in Propionibacterium acnes, a major contributor to wide-spread human disease. *BMC Genomics*, 2013, vol. 14, 620
- **BENAISSA, H. et al.** Engineering of a fluorescent chemogenetic reporter with tunable color for advanced live-cell imaging. *Nat. Commun.*, 2021, vol. 12, 6989
- **PINILLA-REDONDO, R.** ; **RIBER, L.** ; **SORENSEN, S. J.** Fluorescence Recovery Allows the Implementation of a Fluorescence Reporter Gene Platform Applicable for the Detection and Quantification of Horizontal Gene Transfer in Anoxic Environments. *Appl. Environ. Microbiol.*, 2018, vol. 84
- **RANSOM, E. M.** ; **ELLERMEIER, C. D.** ; **WEISS, D. S.** Use of mCherry Red fluorescent protein for studies of protein localization and gene expression in Clostridium difficile. *Appl. Environ. Microbiol*, 2015, vol. 81, 1652-1660
- **CHEN, Y.-J. et al.** Characterization of 582 natural and synthetic terminators and quantification of their design constraints. *Nat. Methods*, 2013, vol. 10, 659-664

- **BABA, T. et al.** Construction of Escherichia coli K-12 in-frame, single-gene knockout mutants: the Keio collection. *Mol. Syst. Biol.*, 2006, vol. 2 (2006), 0008
- **NIELSEN, P. A.** Role of reduced sulfur compounds in nutrition of Propionibacterium acnes. *J. Clin. Microbiol.*, 1983, vol. 17, 276-279
- **MARK, H.** ; **HARDING, C. R.** Amino acid composition, including key derivatives of eccrine sweat: potential biomarkers of certain atopic skin conditions. *Int. J. Cosmet. Sci.*, 2013, vol. 35, 163-168
- **KIM, N. M.** ; **SINNOTT, R. W.** ; **SANDOVAL, N. R.** Transcription factor-based biosensors and inducible systems in non-model bacteria: current progress and future directions. *Curr. Opin. Biotechnol.*, 2020, vol. 64, 39-46
- **XIAO, Y.** ; **JIANG, W.** ; **ZHANG, F.** Developing a Genetically Encoded, Cross-Species Biosensor for Detecting Ammonium and Regulating Biosynthesis of Cyanophycin. *ACS Synth. Biol*, 2017, vol. 6, 1807-1815
- **IMMETHUN, C. M. et al.** Oxygen-responsive genetic circuits constructed in Synechocystis sp. PCC 6803. *Biotechnol. Bioeng.*, 2016, vol. 113, 433-442
- **DELORENZO, D. M.** ; **HENSON, W. R.** ; **MOON, T. S.** Development of Chemical and Metabolite Sensors for Rhodococcus opacus PD630. *ACS Synth. Biol.*, 2017, vol. 6, 1973-1978
- **ROTTINGHAUS, A. G.** ; **FERREIRO, A.** ; **FISHBEIN, S. R. S.** ; **DANTAS, G.** ; **MOON, T. S.** Genetically stable CRISPR-based kill switches for engineered microbes. *Nat. Commun.*, 2022, vol. 13, 672
- **ABEDI, M. H. et al.** Ultrasound-controllable engineered bacteria for cancer immunotherapy. *Nat. Commun.*, 2022, vol. 13, 1585
- **DAS GUPTA, T.** ; **BANDYOPADHYAY, B.** ; **DAS GUPTA, S. K.** Modulation of DNA-binding activity of Mycobacterium tuberculosis HspR by chaperones. *Microbiology*, 2008, vol. 154, 484-490
- **SPOHN, G.** ; **SCARLATO, V.** The autoregulatory HspR repressor protein governs chaperone gene transcription in Helicobacter pylori. *Mol. Microbiol.*, 1999, vol. 34, 663-674
- **GEORGI, C.** ; **BUERGER, J.** ; **HILLEN, W.** ; **BERENS, C.** Promoter strength driving TetR determines the regulatory properties of Tet-controlled expression systems. *PLoS One*, 2012, vol. 7, e41620
- **D'ORAZIO, M. et al.** Regulatory and structural properties differentiating the chromosomal and the bacteriophage-associated Escherichia coli O157:H7 Cu, Zn superoxide dismutases. *BMC Microbiol.*, 2008, vol. 8, 166
- **HOLLAND, C. et al.** Proteomic identification of secreted proteins of Propionibacterium acnes. *BMC Microbiol.*, 2010, vol. 10, 230

- **ALLHORN, M.** ; **ARVE, S.** ; **BRÜGGEMANN, H.** ; **LOOD, R.** A novel enzyme with antioxidant capacity produced by the ubiquitous skin colonizer Propionibacterium acnes. *Sci. Rep.*, 2016, vol. 6, 36412
- **SMITS, J. P. H. et al.** Immortalized N/TERT keratinocytes as an alternative cell source in 3D human epidermal models. *Sci. Rep.*, 2017, vol. 7, 11838
- **DICKSON, M. A. et al.** Human keratinocytes that express hTERT and also bypass a p16(INK4a)-enforced mechanism that limits life span become immortal yet retain normal growth and differentiation characteristics. *Mol. Cell. Biol.*, 2000, vol. 20, 1436-1447
- **MÜLLER, I. E. et al.** Gene networks that compensate for crosstalk with crosstalk. *Nat. Commun.*, 2019, vol. 10, 4028
- **INDA-WEBB, M. E. et al.** Sub-1.4 cm3 capsule for detecting labile inflammatory biomarkers in situ. *Nature*, 2023, vol. 620, 386-392
- **SÖRENSEN, M. et al.** Mutagenesis of Propionibacterium acnes and analysis of two CAMP factor knockout mutants. *J. Microbiol. Methods*, 2010, vol. 83, 211-216
- **PRONOBIS, M. I.** ; **DEUITCH, N.** ; **PEIFER, M.** The Miraprep: A Protocol that Uses a Miniprep Kit and Provides Maxiprep Yields. *PLoS One*, 2016, vol. 11, e0160509
- **HAHNE, F. et al.** flowCore: a Bioconductor package for high throughput flow cytometry. *BMC Bioinformatics*, 2009, vol. 10, 106
- Organotypic 3D Skin Models: Human Epidermal Equivalent Cultures from Primary Keratinocytes and Immortalized Keratinocyte Cell Lines. **RIKKEN, G.** ; **NIEHUES, H.** ; **VAN DEN BOGAARD, E. H.** Molecular Dermatology: Methods and Protocols. Springer US, 2020, 45-61
- **EWELS, P. A. et al.** The nf-core framework for community-curated bioinformatics pipelines. *Nat. Biotechnol.*, 2020, vol. 38, 276-278
- **PATEL, H. et al.** *Nf-Corelrnaseq: Nf-Core/rnaseq v3.0 - Silver Shark*, 2020
- **DI TOMMASO, P. et al.** Nextflow enables reproducible computational workflows. *Nat. Biotechnol.*, 2017, vol. 35, 316-319
- **DOBIN, A. et al.** STAR: ultrafast universal RNA-seq aligner. *Bioinformatics*, 2013, vol. 29, 15-21
- **LI, H. et al.** The Sequence Alignment/Map format and SAMtools. *Bioinformatics*, 2009, vol. 25, 2078-2079
- **LI, H.** A statistical framework for SNP calling, mutation discovery, association mapping and population genetical parameter estimation from sequencing data. *Bioinformatics*, 2011, vol. 27, 2987-2993
- **LOVE, M. I.** ; **HUBER, W.** ; **ANDERS, S.** Moderated estimation of fold change and dispersion for RNA-seq data with DESeq2. *Genome Biol*, 2014, vol. 15, 550
- **LIVAK, K. J.** ; **SCHMITTGEN, T. D.** Analysis of relative gene expression data using real-time quantitative PCR and the 2(-Delta Delta C(T)) Method. *Methods*, 2001, vol. 25, 402-408